(19) Europäisches Patentamt
European Patent Office
Office européen des brevets

(11) Veröffentlichungsnummer: **0 011 859**
**B1**

(12) # EUROPÄISCHE PATENTSCHRIFT

(45) Veröffentlichungstag der Patentschrift: **22.12.82**

(51) Int. Cl.³: **C 07 D 493/10,** C 12 P 17/02, A 23 K 1/17, A 61 K 31/35 // (C07D493/10, 307/00, 311/00)

(21) Anmeldenummer: **79104744.2**

(22) Anmeldetag: **28.11.79**

(54) **Urethan-Derivate und deren Salze, die Herstellung dieser Verbindungen, sowie entsprechende Präparate.**

(30) Priorität: **29.11.78 US 964564**
**05.09.79 US 72818**
**20.09.79 US 77415**

(43) Veröffentlichungstag der Anmeldung: **11.06.80 Patentblatt 80/12**

(45) Bekanntmachung des Hinweises auf die Patenterteilung: **22.12.82 Patentblatt 82/51**

(84) Benannte Vertragsstaaten: **AT BE CH DE FR IT LU NL SE**

(56) Entgegenhaltungen:
**DE-A-2 711 953**
**DE-A-2 712 048**
**FR-A-2 169 201**
**US-A-3 501 568**
**US-A-3 839 557**
**US-A-3 995 027**
**US-A-4 027 034**
**US-A-4 058 620**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Liu, Chao-Min, 36 Rockledge Place, Cedar Grove, N.J. 07009 (US)**
Erfinder: **Westley, John, 91 South Mountain Avenue, Cedar Grove, N.J. 07009 (US)**

(74) Vertreter: **Wuesthoff, Franz, Dr.-Ing. et al, Patentanwälte Wuesthoff -v. Pechmann-Behrens-Goetz Schweigerstrasse 2, D-8000 München 90 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Urethan-Derivate und deren Salze, die Herstellung dieser Verbindungen sowie entsprechende Präparate

Die vorliegende Erfindung betrifft Urethan-Derivate der allgemeinen Formel

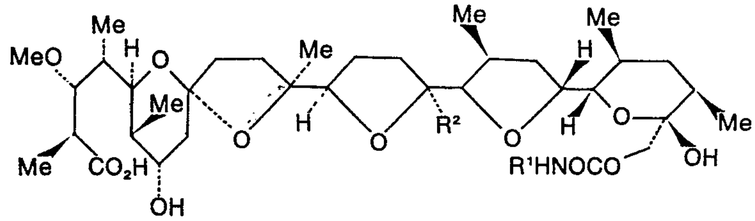

I

in der

$R^1$ Phenyl, $C_{1-7}$-Alkyl-Phenyl, Halogen-Phenyl, Nitro-Phenyl, Phenoxy-Phenyl, $C_{1-7}$-Alkyl, $C_{3-7}$-Cycloalkyl, Phenyl-$C_{1-7}$-Alkyl, $C_{1-7}$-Alkanoyl-Phenyl, $C_{1-7}$-Alkoxy-Phenyl oder Phenyl-$C_{3-7}$-Cycloalkyl und

$R^2$ Methyl oder Äthyl darstellen, und deren pharmazeutisch verträgliche Salze.

Das in Formel I verwendete Symbol Me stellt die Methylgruppe dar.

Die erfindungsgemässen Verbindungen und ihre Salze sind wirksam als antibakterielle Mittel und fördern das Wachstum von Wiederkäuern und monogastrischen Tieren, welche faseriges Pflanzenmaterial im Blinddarm vergären; ferner sind sie coccidiostatische Mittel sowie Mittel gegen erhöhten Blutdruck, Malaria und Schweinedysenterie.

Der Ausdruck «Alkanoyl» bedeutet einen $C_1$–$C_7$-vorzugsweise $C_1$–$C_4$-Alkancarbonsäurerest, d.h. ein Radikal der Formel

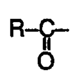

in der

R $C_1$–$C_6$-Alkyl oder Wasserstoff darstellt, z.B. Acetyl, Propionyl, Butyryl und dergleichen.

Der Ausdruck «Alkoxy» bedeutet eine $C_1$–$C_7$ niedere Alkylgruppe mit einer Sauerstoffunktion, wie beispielsweise Methoxy, Äthoxy, n-Propoxy und dergleichen.

Ein Substituent am Phenylkern steht vorzugsweise in 4-Stellung, wie z.B. in 4-Alkylaryl, z.B. 4-Methyl-Phenyl (4. Tolyl), 4-Halogenphenyl, z.B. 4-Chlorphenyl.

Der Ausdruck «Alkyl» bedeutet einen $C_1$–$C_7$ geradkettigen oder verzweigten, insbesondere einen $C_1$–$C_4$-Kohlenwasserstoffrest, wie z.B. Methyl, Äthyl, n-Propyl, Isopropyl, n-Butyl. Die «Alkyl»-Gruppe kann durch eine Phenylgruppe substituiert sein, wobei eine «Phenylalkyl»-Gruppe erhalten wird, z.B. 1-Phenyläthyl, 2-Phenyläthyl (Phenäthyl).

Der Ausdruck «Cycloalkyl» bedeutet cyclische Kohlenwasserstoffgruppen mit 3–7 Kohlenstoffatomen, wie z.B. Cyclopropyl, Cyclobutyl, Cyclohexyl und dergleichen, wobei Cyclohexyl bevorzugt ist. Die «Cycloalkyl»-Gruppe kann durch eine Phenyl-Gruppe substituiert sein, wobei eine «Phenylcycloalkyl»-Gruppe entsteht, z.B. 2-(Phenyl)-cyclopropyl.

Gewisse der Urethan-Derivate der Formel I, und zwar worin $R^1$ Phenäthyl darstellt, und ihre pharmazeutisch verträglichen Salze werden durch Mikroorganismen der Gattung Streptomyces mit der Stammbezeichnung X-14667, X-14573 und X-14575 erzeugt. Streptomyces sp. X-14667 wurde aus einer Bodenprobe vom Aesculapius-Tempel, Epidaurus, Griechenland, isoliert. Streptomyces sp. X-14573 wurde aus einer Bodenprobe bei der Universität von Arizona, Tempe, Arizona, isoliert. Streptomyces sp. X-14575 wurde aus einer Bodenprobe in einem Maisfeld in Catskills, New York, isoliert. Streptomyces sp. X-14667, X-14573 und X-14575 wurden in der Mikroorganismensammlung des United States Department für Landwirtschaft, Agricultural Research Service, Northern Region Research Laboratories (NRRL), Peoria, Illinois, USA, als gefriergetrocknete Proben deponiert. Die Kulturen wurden bei NRRL am 29. Juni 1978 unter den Identifizierungsnummern NRRL 11336 (X-14667), NRRL 11337 (X-14573) und NRRL 11338 (X-14575) deponiert. Subkulturen davon wurden am 23. August 1979 als gefriergetrocknete Proben bei American Type Culture Collection, Rockville, Maryland, USA, unter den Identifizierungsnummern ATCC 31551 (X-14667), ATCC 31552 (X-14573) und ATCC 31553 (X-14575) deponiert.

Die erfindungsgemässen Urethan-Derivate sind Polyätherantibiotika und bilden eine Vielzahl pharmazeutisch verträglicher Salze. Diese Salze werden nach an sich bekannten Methoden aus der freien Säureform der Antibiotika hergestellt, beispielsweise durch Umsetzen der freien Säure in Lösung mit einer geeigneten Base bzw. einem geeigneten Salz. Beispiele solcher pharmazeutisch verträglichen basischen, salzbildenden Stoffe sind Alkalimetallbasen, wie Natriumhydroxid, Kaliumhydroxid, Lithiumhydroxid und dergleichen; Erdalkalimetallbasen, wie z.B. Calciumhydroxid, Bariumhydroxid und dergleichen; und Ammoniumhydroxid. Alkali- oder Erdalkalimetallsalze, welche zur Herstellung von pharmazeutisch verträglichen Salzen der Urethan-Derivate der Formel I verwendet werden können, sind z.B. Carbonate, Bicarbonate und Sulfate.

Beispiele organischer Basen, welche mit den erfindungsgemässen Urethan-Derivate pharmazeutisch verträgliche Salze bilden können, sind

niedere Alkylamine oder primäre, sekundäre oder tertiäre Hydroxy-niederalkylamine, wie z. B. Äthylamin, Isopropylamin, Diäthylamin, Methyl-n-butylamin, Äthanolamin und Diäthanolamin.

Ein besonders bevorzugtes Amin ist N-Methylglucamin. Salze von N-Methylglucamin sind wegen ihrer Wasserlöslichkeit von besonderem Wert, wodurch die Verbindungen zur parenteralen Verwendung eingesetzt werden können.

Morphologische Merkmale

Repräsentative Stämme von Streptomyces sp. X-14667, X-14573 und X-14575 besitzen folgende Merkmale:

Natriumchloridtolerenz, Hydrolyse von Casein und Reduktion von Nitrat wurden durch die Methoden von Gordon und Smith, J. Bacteriol., 66, 41–48, 1953, bestimmt. Hydrolyse von Stärke wurde nach Wachstum auf Agar mit Actinomyces-Medium (Difco) mit 0,25% löslicher Stärke bestimmt. Der Test wurde durch Überschichtung der Schalen mit Jod-Kaliumjodidlösung durchgeführt. Gelatinehydrolyse wurde gemäss Skerman (A Guide to Identification of the Genera of Bacteria, The Williams and Wilkins Co., Baltimore, 1967) getestet unter Verwendung von Actinomyces-Brühe (Difco) mit 2% Agar anstelle von Fleischinfusions-Agar. Alle Versuche wurden bei 28 °C durchgeführt.

Zur Charakterisierung von Wachstum und Pigmentierung wurden die standardisierten ISP-Medien von Shirling und Gottlieb verwendet (die Farbe wurde nach 2wöchiger Bebrütung bei 28 °C bestimmt). Auch Kohlenstoffverwertung wurde durch die Methode von Shirling und Gottlieb bestimmt (Int. J. Syst. Bacteriol., 16, 313–340, 1966). Zur Beimpfung wurde eine 24 Stunden alte ISP-1-Kulturbrühe homogenisiert und zentrifugiert zwecks Erhalts einer gewaschenen Suspension. Die Fähigkeit des Organismus, bei 10, 28, 37, 45 und 50 °C zu wachsen, wurde durch Beimpfung an ISP-1(Difco)-Medium untersucht. Zellwandanalyse wurde durch die Methode von Becker et al. untersucht (Applied Microbiol. 12, 421–423, 1964).

Mikroskopische Untersuchung. Streptomyces sp. X-14667, X-14573 und X-14575 erzeugen ein Substratmycel, welches in Sporen nicht fragmentiert, und ein Luftmycel, welches später Sporenketten bildet. Nach 14tägiger Bebrütung bei 28 °C besitzen die Sporenketten eine Rectusflexibilis-Form mit mehr als 50 Sporen pro Kette. Die Sporen sind glatt, mit einem Grössenbereich von 0,70 × 0,5 µm bis 1,35 × 0,38 µm für X-14667; 0,7 × 0,4 bis 0,45 × 0,4 µm für X-14573; 0,8 × 0,5 µm bis 0,6 × 1,0 µm für X-14575.

Die Zellwände der Kulturen enthalten das LL-Isomere von Diaminopimelinsäure. Dieses Merkmal ordnet die Mikroorganismen in die Gattung Streptomyces ein (Lechevalier et al., Adv. Appl. Microbiol., 14, 47–72, 1971).

Makroskopische Untersuchung. Das Wachstumsverhalten, der Sporulationsgrad, die Farbe der Sporen sowie die Farbe der Rückseite des Mycels und die Anwesenheit von durch Streptomyces sp. X-14667, X-14573 und X-14575 auf verschiedenen festen Nährmedien erzeugtem löslichem Pigment werden in den nachstehenden Tabellen 1, 2 und 3 zusammengefasst.

Tabelle 1
Kulturmerkmale von Streptomyces sp. X-14667

| Agar-Medium | Wachstum; Sporulationsgrad | Farbe der Sporen[a] | Farbe der Rückseite des Mycels[a] |
|---|---|---|---|
| Hefe/Malz-Extrakt (ISP-2)[b] | mittleres Wachstum; mittlere Sporulation | b (austernweiss) | 31g (ziegelbraun) |
| Hafermehl (ISP-3)[b] | mittleres Wachstum; mittlere Sporulation; leicht rosafarbene Rückseite, welche mit NaOH in blau übergeht | b (austernweiss) | 5ge (rosenrot) |
| Anorganische Salze (Stärke ISP-4)[b] | reichliches Wachstum; reichliche Sporulation; hydrolysiert Stärke | 3cb (sandfarben) | 5ie (kupferfarben) |
| Glycerin/Asparagin (ISP-5)[b] | mässiges Wachstum; reichliche Sporulation | b (austernweiss) | 31g (ziegelbraun); 2ec (biskuitfarben) am Rande |
| Czapek-Dox[c] | reichliches Wachstum; reichliche Sporulation | 3dc (naturfarben) | 2gc (bambusfarben); 2ec (biskuitfarben) am Rande |

[a] Die Farbskala wurde Color Harmony Manual, 4th ed., 1958 (Container Corporation of America, Chicago), entnommen.
[b] Medien empfohlen durch das Internationale Streptomycesprojekt (Shirling, E.B. & D. Gottlieb, Int. J. Syst. Bacteriol. 16, 313–340, 1966).
[c] Czapek-Dox-Brühe (BBL) mit einem Zusatz von 1,5% Agar.

Tabelle 2 Kulturmerkmale von Streptomyces sp. X-14573

| Agar-Medium | Wachstum; Sporulationsgrad | Farbe der Sporen[a] | Farbe der Rückseite des Mycels[a] |
|---|---|---|---|
| Hefe/Malz-Extrakt (ISP-2)[b] | reichliches Wachstum; gute Sporulation; dunkles rotbraunes lösliches Pigment | 3cb (sandfarben) | 5nl (schokoladefarben) |
| Hafermehl (IPS-3)[b] | mässiges bis reichliches Wachstum; mässige Sporulation | b (austernweiss) | 4ie (korkfarben) |
| Anorganische Salze (Stärke ISP-4)[b] | reichliches Wachstum; gute Sporulation; hydrolysiert Stärke; braunes lösliches Pigment | 5cb (keine Bezeichnung) | 4nl (schokoladefarben) |
| Glycerin/Asparagin (ISP-5)[b] | reichliches Wachstum; gute Sporulation; braunes lösliches Pigment; hygroskopisch | 3dc (naturfarben) | 4nl (schokoladefarben) |
| Czapek-Dox[c] | mässiges bis reichliches Wachstum; gute Sporulation | 3cb (sandfarben) | 4ie (korkfarben) |

[a] Die Farbskala wurde Color Harmony Manual, 4th ed., 1958 (Container Corporation of America, Chicago), entnommen.

[b] Medien empfohlen durch das Internationale Streptomycesprojekt (Shirling, E.B. & D. Gottlieb, Int. J. Syst. Bacteriol. 16, 313–340, 1966).

[c] Czapek-Dox-Brühe (BBL) mit einem Zusatz von 1,5% Agar.

Tabelle 3 Kulturmerkmale von Streptomyces sp. X-14575

| Agar-Medium | Wachstum; Sporulationsgrad | Farbe der Sporen[a] | Farbe der Rückseite des Mycels[a] |
|---|---|---|---|
| Hefe/Malz-Extrakt (ISP-2)[b] | mässiges Wachstum; spärliche Sporulation; braunes lösliches Pigment | b (austernweiss) | 4nl (schokoladefarben) |
| Hafermehl (ISP-3)[b] | mässiges Wachstum; mässige Sporulation | b (austernweiss) | zur Hauptsache 3ec (Farbe von unglasiertem Porzellan); punktweise 31g (ziegelbraun) |
| Anorganische Salze (Stärke ISP-4)[b] | mässiges Wachstum; mässige Sporulation; hydrolysiert Stärke; bräunliches, lösliches Pigment, wo keine Hydrolyse | b (austernweiss) | 31g (ziegelbraun) |
| Glycerin/Asparagin (ISP-5)[b] | mässiges Wachstum; keine Sporulation | 31g (ziegelbraun); 3ie (kamelfarben) an isolierten Rändern; keine Sporulation | 3ie (kamelfarben) |
| Czapek-Dox[c] | mässiges Wachstum; spärliche Sporulation | 41g (hell gewürzbraun) meistens, wo keine Sporulation; b (austernweiss), wo fleckenweise Sporulation | 4ie (korkfarben) |

[a] Die Farbskala wurde Color Harmony Manual, 4th ed., 1958 (Container Corporation of America, Chicago), entnommen.

[b] Medien empfohlen durch das Internationale Streptomycesprojekt (Shirling, E.B. & D. Gottlieb, Int. J. Syst. Bacteriol. 16, 313–340, 1966).

[c] Czapek-Dox-Brühe (BBL) mit einem Zusatz von 1,5% Agar.

Physiologische Kennzeichen. Die Kohlenstoffverwertung und die Stoffwechselkennzeichen von Streptomyces sp. X14667, X-14573 und X-14575 werden in den nachstehenden Tabellen 4 und 5 mit dem Monensin erzeugenden Stamm S. cinnamonensis 1712A vergleichen.

Tabelle 4. Vergleiche der Kohlenstoffverwertung von Streptomyces sp. X-14667, X-14573 und X-14575 mit S. cinnamonesis 1712A.

| | Wachstum von: | | | |
|---|---|---|---|---|
| Kohlenstoffquelle | X-14667 | X-14573 | X-14575 | S. cinnamonensis 1712A |
| D-Glucose | ++ | ++ | ++ | ++ |
| D-Xylose | + | ++ | ++ | ++ |
| L-Arabinose | ++ | ++ | ++ | +(+) |
| L-Rhamnose | – | ++ | – | ++ |
| D-Fructose | +(+) | ++ | ++ | ++ |
| D-Galactose | ++ | ++ | ++ | ++ |
| Raffinose | ++ | +(+) | ++ | +(+) |
| D-Mannit | ++ | ++ | ++ | ++ |
| i-Inosit | + | ++ | ++ | ++ |
| Salicin | ± bis + | ++ | ++ | +(+) |
| Saccharose | – | – | – | – |
| Cellulose | – | – | – | – |

– Negative Reaktion; ± zweifelhafte Reaktion; + kräftigeres Wachstum als auf Kontrollprobe, aber weniger als auf Clucose; ++ positive Reaktion, gleichwertig mit dem Wachstum auf Glucose.

Tabelle 5
Stoffwechselmerkmale

| Test | X-14667 | X-14573 | X-14575 | S. cinnamonensis 1712A |
|---|---|---|---|---|
| ISP-6 Verdunkelung | – | – | – | – |
| Melanin, ISP-7 | – | – | – | – |
| Casein-Hydrolyse | + | + | + | + |
| Gelatine-Hydrolyse | + | + | + | + |
| Stärke-Hydrolyse | + | + | + | + |
| NaCl-Toleranz (%) | <10, wahrscheinlich = 7 | 7 | 7 | <10 |
| Temperaturbereich des Wachstums, °C | 10–37 | | | 10–37 |
| ISP-1 Verdunkelung | – | +, redwood | ± leicht bräunlich | + leicht |
| Pigment der Rückseite | leicht rot-braun | braun | braun gelegentlich | bräunlich, gelegentlich |
| Lösliches Pigment | rosafarben; mit NaOH umwandelbar in blau | rot-braun | braun | bräunlich |
| Erzeugung von Antibiotikum | Monensin A und B sowie die entsprechenden Phenäthylurethane | | | Monensin A und B |
| Nitrat-Reduktion | + | ± | + | ± in 2 Wochen |
| Hygroskopische Eigenschaften | + | leicht auf ISP-5 | – | – |

Ein Vergleich der Beschreibung von Streptomyces sp. X-14667, X-14573 und X-14575 mit den in Bergeys Manual beschriebenen Streptomyces-Spezies (Buchanan and Gibbons, Bergeys Manual of Determinative Bacteriology, 8. Auflage, 748–829, 1974), H. Nonomura's Schlüssel zur Klassifizierung (J. Ferment. Technol., 52, 78–92, 1974) sowie die Klassifizierung von Pridham und Lyons (Dev. Ind. Microbiol. 10, 183–221, 1969) zeigte S. cinnamonensis als der ähnlichste Verwandte zu den obigen Stämmen aufgrund der Kombination folgender Kriterien: graue Farbe der Sporen, Rectusflexibilis-Form der Sporenketten, glatte Sporenoberfläche, chromogene Reaktion auf ISP-Medien 1 und 6 sowie die Kohlenstoffverwertung. S. cinnamonensis ähnelt X-14667, X-14573 und X-14575, indem sie alle Monensin A und Monensin B erzeugen. S. cinnamonensis unterscheidet sich jedoch von den erwähnten Organismen durch die fehlende Fähigkeit, die Monensinphenäthylurethane zu erzeugen.

Die hier beschriebenen Streptomyces-Spezies X-14667, X-14573 und X-14575 schliessen sämtliche Stämme von Streptomyces ein, welche die erfindungsgemässen Monensinphenäthylurethane erzeugen, und welche nicht eindeutig von den Stämmen NRRL 11336 (ATCC 31551), NRRL 11337 (ATCC 31552) und NRRL 11338 (ATCC 31553) und deren Subkulturen, einschliesslich Mutanten und Varianten davon, unterschieden werden können. Mit Hilfe der hier beanspruchten Verbindungen bietet die Differenzierung der diese Verbindungen erzeugenden Stämme von anderen dem Fachmann keine Schwierigkeiten.

Streptomyces X-14667, X-14573 und X-14575 erzeugen nach Züchtung unter geeigneten Bedingungen Monensinurethan-Derivate. Die dafür benötigte Gärlösung wird durch Beimpfung des Nährmediums mit Sporen oder Mycel des Mikroorganismus hergestellt; die Nährlösung wird anschliessend unter aeroben Bedingungen kultiviert. Es ist ebenfalls möglich, Kultivierung an einem festen Nährboden durchzuführen. Für die Erzeugung grösserer Mengen wird jedoch ein flüssiges Medium bevorzugt. Die Gärtemperatur kann im weiten Bereich von 20–35 °C variiert werden, jedoch sind Temperaturen von 26–30 °C und im wesentlichen neutrale pH bevorzugt. Für die submerse aerobe Fermentation kann das Nährmedium als Kohlenstoffquelle ein im Handel erhältliches Glyceridöl oder ein Kohlenhydrat, wie z.B. Glycerin, Glucose, Maltose, Lactose, Dextrin, Stärke usw., in reinem oder rohem Zustand enthalten; als Stickstoffquelle kommen organische Materialien, wie Soyabohnenmehl, lösliche Rückstände aus der Brennerei, Erdnussmehl, Baumwollsamenmehl, Fleischextrakt, Pepton, Fischmehl, Hefeextrakt, Maisquellwasser usw., in Betracht. Erwünschtenfalls können auch anorganische Stickstoffquellen verwendet werden, wie z.B. Nitrat und Ammoniumsalze; Mineralsalze, wie beispielsweise Ammoniumsulfat, Magnesiumsulfat, Natriumchlorid, Kaliumchlorid, Kaliumphosphat, Calciumcarbonat und Spurenmengen von Schwermetallsalzen; Puffer, wie Natriumcitrat, Calciumcarbonat oder -phosphate. Wenn die Fermentation unter belüfteten, submersen Bedingungen durchgeführt wird, verwendet man vorzugsweise ein Schaumbekämpfungsmittel, wie flüssiges Paraffin, fettes Öl oder Siliconprodukte. Mehr als eine Art von Kohlenstoffquelle, Stickstoffquelle bzw. Schaumbekämpfungsmittel können verwendet werden.

Die antibiotische Wirksamkeit der erfindungsgemässen Urethan-Derivate wird durch die nachstehende Tabelle 6 veranschaulicht.

Aus der in Tabelle 6 angegebenen in-vitro-Wirkung der erfindungsgemässen Verbindungen gegen gewisse grampositive Bakterien kann man schliessen, dass die Verbindungen als antibakterielle Mittel in Waschlösungen für sanitäre Zwecke, wie z.B. zum Händewaschen und zur Reinigung von Ausrüstungen, Böden bzw. Einrichtungen von kontaminierten Räumen oder Laboratorien, verwendbar sind.

Es wurde ferner gefunden, dass gewisse der erfindungsgemässen Urethan-Derivate, d.h. diejenigen der Formel I, worin $R^2$ Aryl, Halogenaryl, Nitroaryl, Alkylaryl oder Cycloalkyl darstellt, sowie ihre pharmazeutisch verträglichen Salze eine Anti-Coccidiosewirkung gegen den Mikroorganismus Eimeria tenella ausüben.

Diese Anti-Coccidiosewirkung wird an Laborküken wie folgt veranschaulicht:

Versuchsmethode. – Es werden 10 Küken pro Dosis eingesetzt. 10 Küken werden als Gewichtskontrolle und 10 Küken als infizierte Kontrolle verwendet. Die Prüfsubstanz wird 48 Stunden vor der Infektion gegeben. 1 g der Prüfsubstanz wird in einem mechanischen Mixer mit der benötigten Menge Kükenfutter vermischt, um die gewünschte Dosierung zu erreichen. Die Infektion besteht aus etwa 200 000 Oocysten, peroral mit Hilfe einer Pipette verabreicht. Der Versuch dauert 11 Tage, wonach die überlebenden Tiere in der Autopsie auf grobe caecale Verletzungen untersucht werden. Die Zahl der überlebenden Tiere und die Zahl der caecalen Verletzungen werden ermittelt. Die Ergebnisse werden als «durchschnittlicher Infektionsgrad» ausgedrückt. Bei einem durchschnittlichen Infektionsgrad von weniger als 2,5 wird die zugehörige Dosis als signifikant wirksam angesehen.

Tabelle 6
Antimikrobielle Spektren von Urethan-Derivaten von Monensin
Mindesthemmkonzentration (mcg/ml)[a]

| | Staph. aureus 6538 | Sarcina lutea 9341 | Strepto coccus faecium 8043 | Bacillus sp. E 27859 | Bacillus subtilis 583[b] | Bacillus mega-terium 8011 | Bacillus sp. TA 27860 | Myco-bact. phlei 355 | Strepto-coccus cellul. 3313 | Paecilo-myces varioti 25820 |
|---|---|---|---|---|---|---|---|---|---|---|
| Monensin B, Phenäthylurethan (hergestellt durch Fermentation) | 6,3 | 3,1 | 0,8 | 1,6 | 3,1 | 3,1 | 3,1 | 25 | 12,5 | – |
| Monensin A, Phenäthylurethan (hergestellt durch Fermentation) | 6,3 | 1,6 | 0,4 | 0,8 | 1,6 | 0,8 | 1,6 | 6,3 | 12,5 | 12,5 |
| Monensin B, (R)-1-Phenyläthylurethan | 6,3 | 3,1 | 0,8 | 0,4 | 1,6 | 1,6 | 1,6 | 12,5 | 3,1 | 3,1 |
| Monensin B, (S)-1-Phenyläthylurethan | 1,6 | 1,6 | 0,2 | 0,1 | 0,8 | 0,4 | 0,2 | 6,3 | 6,3 | 6,3 |
| Monensin A, 4-Bromphenylurethan | 0,8 | 0,2 | 0,1 | 0,05 | 0,4 | 0,1 | 0,2 | 1,6 | 0,8 | 1,6 |
| Monensin A, 4-Chlorphenylurethan | 0,4 | 0,2 | 0,05 | 0,02 | 0,4 | 0,2 | 0,2 | 0,8 | 0,8 | 3,1 |
| Monensin A, Phenylurethan | 0,8 | 0,8 | 0,1 | 0,05 | 0,4 | 0,4 | 0,4 | 3,1 | 3,1 | 3,1 |
| Monensin A, 4-Fluorphenylurethan | 1,6 | 0,8 | 0,1 | 0,2 | 0,8 | 0,4 | 0,8 | 3,1 | 3,1 | 3,1 |
| Monensin A, Cyclohexylurethan | 1,6 | 0,8 | 0,1 | 0,2 | 0,8 | 0,4 | 0,8 | 3,1 | 3,1 | 3,1 |
| Monensin A, 2-(Phenyl)-cyclopropylurethan | 3,1 | 0,8 | 0,2 | 0,4 | 0,8 | 0,8 | 0,4 | 3,1 | 12,5 | 6,3 |
| Monensin A, Methylurethan | 25 | 25 | 1,6 | 6,3 | 1,6 | 6,3 | 12,5 | – | – | – |
| Monensin A, n-Butylurethan | 1,6 | 1,6 | 0,2 | 0,2 | 0,4 | 0,8 | 0,4 | 6,3 | 3,1 | 6,3 |
| Monensin A, 4-(Methylcarbonyl)-phenylurethan | 1,6 | 1,6 | 0,8 | 0,2 | 1,6 | 1,6 | 0,8 | 6,3 | 3,1 | 6,3 |
| Monensin A, 4-Phenoxyphenylurethan | 0,4 | 0,4 | 0,1 | 0,4 | 0,4 | 0,4 | 0,2 | 1,6 | 0,8 | 0,5 |
| Monensin A, 4-Jodphenylurethan | 0,4 | 0,2 | 0,1 | 0,02 | 0,4 | 0,2 | 0,2 | 1,6 | 0,4 | 1,6 |
| Monensin A, 4-Methoxyphenylurethan | 1,6 | 1,6 | 0,4 | 0,1 | 0,8 | 0,8 | 0,8 | 6,3 | 1,6 | 6,3 |
| Monensin A, 4-Methylphenylurethan | 0,4 | 0,4 | 0,2 | 0,05 | 0,4 | 0,2 | 0,2 | 1,6 | 0,8 | 1,6 |

[a] Niedrigste zweifache Verdünnung mit einer Hemmzone im Agardiffusionstest.
[b] NRRL Deponierungsnummer, die verbleibenden Nummern sind ATCC-Nummern.

Tabelle 7
Anti-Coccidiose-Wirkung von Monensinurethanen, welche dem Futter von mit Eimeria tenella infizierten Küken zugegeben wurden

| Verbindung | Dosierung in Futter, ppm | Durchschnittlicher Infektionsgrad |
|---|---|---|
| Uninfizierte, unbehandelte Kontrolle | keine | 0,0 |
| Infizierte, unbehandelte Kontrolle | keine | 3,0 |
| Monensin A, 4-Nitrophenylurethan | 75 | 0,5 |
| Monensin A, 4-Bromphenylurethan | 75 | 0,7 |
| Monensin A, Phenylurethan | 75 | 0,9 |
| Monensin A, 4-Chlorphenylurethan | 65 | 0,8 |
| Monensin A, 4-Methylphenylurethan | 75 | 1,1 |
| Monensin A, 4-Jodphenylurethan | 125 | 0,1 |
| Monensin A, 4-Fluorphenylurethan | 125 | 1,0 |
| Monensin A, Cyclohexylurethan | 125 | 0,8 |

Die erfindungsgemässen Urethan-Derivate sind ebenfalls gegen Treponema hyodysenteriae wirksam. Die entsprechenden Mindesthemmkonzentrationen der Urethan-Derivate sind wie folgt:

| Verbindung | Mindesthemmkonzentration (mcg/ml) |
|---|---|
| Monensin B, Phenäthylurethan (hergestellt durch Fermentation) | 0,4–2,0 |
| Monensin B, Phenäthylurethan (synthetisches Produkt) | 2,0 |
| Monensin A, 4-Bromphenylurethan | 0,4 |
| Monensin B, (R)-1-Phenyläthylurethan | 0,4 |
| Monensin A, Phenäthylurethan | 0,4 |
| Monensin A, Phenylurethan | 0,08–0,4 |
| Monensin A, 4-Chlorphenylurethan | 0,08 |
| Monensin B, (S)-1-Phenyläthylurethan | 0,4 |
| Monensin A, Cyclohexylurethan | 0,4 |
| Monensin A, 2-(Phenyl)-cyclopropylurethan | 0,4 |
| Monensin A, 4-Fluorphenylurethan | 0,08 |

Die Versuchsmethodik zur Ermittlung von Wirksamkeit gegen Treponema hyodysenteriae, ein Erreger von Schweinedysenterie, besteht im Beimpfen von Blutagarschalen mit einer Serie von 2- oder 4fachen Verdünnungen der Urethan-Derivate mit 10fachen Verdünnungen des T. hyodysenteriae-Stammes. Nach 48stündiger Bebrütung bei 42 °C in einer anaeroben Atmosphäre werden die Mindesthemmkonzentrationen ermittelt als die niedrigste Konzentration einer Verbindung, welche vollständig das am stärksten verdünnte Inoculum eines jeden T. hyodysenteriae-Stammes hemmt.

Die erfindungsgemässen Urethan-Derivate fördern ebenfalls das Wachstum von Wiederkäuern.

Durch Verabreichung der erfindungsgemässen Urethan-Derivate (nachstehend «das Antibiotikum» benannt) wird Ketose verhindert bzw. geheilt; gleichzeitig wird die Futterverwertung verbessert. Die Ursache der Ketose liegt in einer rückständigen Bildung von Propionat. Eine gegenwärtig empfohlene Behandlungsmethode besteht aus Verabreichung von Propionsäure bzw. von Futtermitteln, welche bevorzugt Propionat erzeugen. Es ist offensichtlich, dass die bevorzugte Bildung von Propionat bei der Einnahme von üblichen Futtermitteln die Ketosefälle vermindern wird.

Die einfachste Methode zur Verabreichung des Antibiotikums besteht darin, dass man es mit dem Tierfutter vermischt. Das Antibiotikum kann jedoch auch in anderer Weise verabreicht werden. Es kann beispielsweise zu Tabletten, Tränken, Boli oder Kapseln verarbeitet werden, welche dem Tier in dosierter Form verabreicht werden können. Formulierungen des Antibiotikums in derartigen Dosierungsformen werden nach an sich in der Veterinärpharmazie bekannten Methoden hergestellt.

Kapseln werden in üblicher Weise hergestellt durch Auffüllen von Gelatinekapseln mit dem Antibiotikum in beliebiger Form. Erwünschtenfalls kann das Antibiotikum mit einem inerten festen Verdünnungsmittel verdünnt werden, wie beispielsweise mit einem Zucker, Stärke oder gereinigter kristalliner Cellulose, um das Volumen aus Zweckmässigkeitsgründen zu erhöhen.

Tabletten werden durch herkömmliche pharmazeutische Methoden hergestellt. Eine Tablette enthält im allgemeinen ausser dem Wirkstoff eine Grundlage, einen Desintegrator, ein Absorbens, ein Bindemittel und ein Gleitmittel.

Typische Grundlagen sind z. B. Milchzucker, feiner Puderzucker, Natriumchlorid, Stärke und Mannit. Stärke ist ebenfalls ein guter Desintegrator, ebenso wie Alginsäure. Oberflächenaktive Mittel, wie Natriumlaurylsulfat und Dioctylnatriumsulfosuccinat werden gelegentlich ebenfalls verwendet. Üblicherweise verwendete Absorbentien sind z. B. Stärke und Milchzucker, während Magnesiumcarbonat bei öligen Substanzen Verwendung findet. Oft verwendete Bindemittel sind Gelatine, Gummi arabicum, Stärke, Dextrin und verschiedene Cellulose-Derivate. Häufig verwendete Gleitmittel sind z. B. Magnesiumstearat, Talk, Paraffinwachs, verschiedene Metallseifen und Polyäthylenglykol.

Das Antibiotikum kann ebenfalls in Form eines Bolus mit verzögerter Wirkung verabreicht werden. Derartige Boli werden als Tabletten hergestellt, wobei jedoch ein Mittel zur Verzögerung der Auflösung des Antibiotikums zugefügt wird. Boli sind für Freisetzung während längerer Perioden vorgesehen. Die langsame Auflösung wird durch Wahl einer stark wasserunlöslichen Form des Antibiotikums gewährleistet. Beispielsweise können Eisenfeilspähne zugegeben werden, um die Dichte des Bolus zu erhöhen, damit dieses am Boden des Pansens statisch bleibt.

Auflösung des Antibiotikums wird verzögert durch Anwendung einer Matrize unlöslichen Materials, in welcher der Wirkstoff eingebettet wird. Beispielsweise können pflanzliche Wachse, gereinigte Mineralwachse und wasserunlösliche, polymere Materialien verwendet werden.

Tränke des Antibiotikums werden zweckmässig durch Wahl einer wasserlöslichen Form des Antibiotikums hergestellt. Wird eine unlösliche Form gewünscht, kann eine Suspension hergestellt werden. Wahlweise kann ein Trank als eine Lösung in einem physiologisch einwandfreien Lösungsmittel, wie beispielsweise Polyäthylenglykol, zubereitet werden.

Suspensionen von unlöslichen Formen des Antibiotikums können in Nicht-Lösungsmitteln hergestellt werden, wie beispielsweise in pflanzlichen Ölen, z. B. Erdnuss-, Mais- oder Sesam-Öl, in einem Glykol, wie z. B. Propylenglykol oder Polyäthylenglykol, oder in Wasser, je nach der gewählten Form des Antibiotikums.

Geeignete physiologisch verträgliche Zusätze sind notwendig, um das Antibiotikum in Suspension zu halten. Die Zusätze können Verdickungsmittel sein, wie z. B. Carboxymethylcellulose, Polyvinylpyrrolidon, Gelatine oder Alginate. Verschiedene Klassen von Schaumerzeugern dienen dazu, das Antibiotikum in Suspension zu halten. Beispielsweise sind Lecithin, Alkylphenol/Polyäthylenoxid-Addukte, Naphthalinsulfonate, Alkylbenzolsulfonate und Polyoxyäthylensorbitanester für die Herstellung von Suspensionen in flüssigen Nicht-Lösungsmitteln verwendbar.

Für die Herstellung von Suspensionen können zusätzlich verschiedene Substanzen, welche die Hydrophilität, Dichte und Oberflächenspannung beeinflussen, verwendet werden. Beispiele sind Silicone, Glykole, Sorbit und Zucker, die als Suspendierungsmittel Verwendung finden.

Das Antibiotikum kann dem Verbraucher als eine Suspension oder als ein trockenes Gemisch mit entsprechenden Hilfsmitteln zur Verdünnung vor der Verwendung vorgelegt werden.

Das Antibiotikum kann ebenfalls dem Trinkwasser des Wiederkäuers zugefügt werden. Dabei wird eine wasserlösliche oder wassersuspendierbare Form des Antibiotikums dem Trinkwasser in entsprechender Menge zugegeben. Die Zubereitung des Antibiotikums für das Vermischen mit dem Trinkwasser folgt den gleichen Prinzipien wie die Formulierung von Tränken.

Die zweckmässigste Art, das Tier mit dem Antibiotikum zu behandeln, basiert auf die Formulierung in das Tierfutter. Beliebige Tierfutter können dazu verwendet werden, z. B. übliche Trockenfutter, flüssige Futter und tablettierte Futter. Zur Herstellung eines medikierten Tierfutters wird üblicherweise zuvor eine konzentrierte Vormischung mit dem Antibiotikum hergestellt. Beispielsweise kann eine Vormischung von etwa 2 bis etwa 900 g Antibiotikum pro Kilogramm Vormischung enthalten. Dieser weite Bereich ist durch den benötigten, weiten Konzentrationsbereich für das fertige Futter bedingt. Vormischungen können sowohl in flüssiger als auch in fester Form vorliegen.

Wie oben gezeigt, bewirkt die orale Verabreichung des Antibiotikums eine vorteilhafte Änderung der Erzeugung von Propionat im Verhältnis zu Acetat im Pansen. Es kann deshalb postuliert werden, dass die gleiche Behandlung ebenfalls monogastrische Tiere, die faseriges Pflanzenmaterial im Blinddarm vergären, günstig beeinflussen, denn es kann erwartet werden, dass eine vorteilhafte Veränderung im Propionat/Acetatverhältnis nach oraler Gabe des Antibiotikums auftreten wird. Pferde, Schweine und Kaninchen sind Beispiele von Tieren, welche einen Teil ihres Futters durch Fermentation im Blinddarm verdauen.

Bestimmung der Produktion von flüssigen Fettsäuren beim Rind

Ein Rind, chirurgisch modifiziert durch eine Fistel am Pansen, wird als Quelle der Pansenflüssigkeit verwendet. Die Proben von Pansenflüssigkeit werden via einer Pansenkanüle erhalten. Das Tier wird zweimal täglich mit einer Mischung von 80% Konzentrat (zur Hauptsache gelber Mais mit Zusätzen von Soyabohnenölmehl und Alfalfamehl): 20% Heu gefüttert. Die Pansenflüssigkeit wird vorgängig der morgigen Fütterung genommen. Die Pansenflüssigkeit wird durch 4 Schichten Nesseltuch in einen 4-Liter-Behälter gesiebt und

unter Kohlendioxid aufbewahrt. 2000 ml eiskalte wässrige Pufferlösung folgender Zusammensetzung:

| | | | |
|---|---|---|---|
| $Na_2HPO_4$ | 0,316 g/l | $MgSO_4$ | 0,112 |
| $KH_2PO_4$ | 0,152 | $CaCl_2$ | 0,038 |
| $NaHCO_3$ | 2,260 | $FeSO_4 \cdot 7H_2O$ | 0,008 |
| NaCl | 0,375 | $ZnSO_4 \cdot 7H_2O$ | 0,004 |
| KCl | 0,375 | $CuSO_4 \cdot 5H_2O$ | 0,002 |

werden mit 1000 ml der oben erhaltenen Pansenflüssigkeit versetzt.

Die so gepufferte Pansenflüssigkeit wird in einen 4-Liter-Separiertrichter vorgelegt. Die Flüssigkeit wird mit Kohlendioxid begast, um anaerobe Bedingungen zu gewährleisten.

Erlenmeyer-Kolben zu je 250 ml werden für jede Fermentation verwendet. Jeder Kolben, der mit einer Verbindung versetzt werden soll, erhält 1 g einer Mischung von fein gemahlenem 80% Konzentrat: 20% Alfalfa-Heu. Kolben, welche als Kontrolle verwendet werden sollen, enthalten 1,07 g fein gemahlenes 80% Konzentrat: 20% Alfalfa-Heu. 1 ml einer Lösung der Testsubstanz in einem geeigneten Lösungsmittel wird in jeden Kolben vorgelegt und ½–1 Stunde stehengelassen. Jede Verbindung wird in Doppelproben bei einer Konzentration von 50 ppm untersucht. Lösungsmittel ohne Testverbindung wird in Kontrollkolben vorgelegt. Monensin bei 10 und 50 ppm wird als positive Kontrolle in allen Fermentationsversuchen verwendet.

80 g gepufferte Pansenflüssigkeit werden in jeden Kolben mit Testverbindung vorgelegt, und 85,93 g werden den Kontrollkolben zugegeben. Die Kolben werden jeweils mit Gasauffangverschluss versehen und bei Zimmertemperatur bis zur Fertigstellung aller Kolben stehengelassen.

6 ml Proben werden allen Kontrollkolben entnommen («Nullzeit»-Proben). Die Kolben werden in ein 38 °C Wasserbad vorgelegt; die Bebrütungszeit und das Aufsammeln von Gas aus der Fermentation fangen 10 Minuten später an. Die Kolben werden 4 Stunden unter Schütteln (90 pro Minute) bebrütet.

Die Gasentwicklung jeder Fermentation wird in halbstündigen Intervallen gemessen. Ein dazu geeigneter Apparat wurde durch Trei et al., (J. Anim. Sci., 30, 825, 1970) beschrieben.

Pansenflüssigkeit wird in 25 × 150 mm-Glasröhrchen gegossen und 15 Minuten in einem Eisbad zur Abscheidung von grober Materie stehengelassen. 2 ml 25% (Gewicht pro Volumen) Methaphosphorsäure in 13 ml-Zentrifugenröhrchen werden mit 6 ml Pansenflüssigkeit versetzt. Jedes Röhrchen wird verschlossen und gründlich gemischt. Die Röhrchen werden in einem Eisbad 30 Minuten stehengelassen und anschliessend 10 Minuten bei 16 000 Upm zentrifugiert. 4 ml der überstehenden Lösung werden anschliessend mit 1 ml internem Standard (0,25% 2-Methylvaleriansäure) versetzt. Das erhaltene Gemisch wird durch ein 0,22-µ-Filter mit Hilfe einer 5-ml-Spritze filtriert. Das Filtrat wird in 1-ml-Glasröhrchen mit Teflonverschluss versiegelt.

Jedes Röhrchen aus den verschiedenen Fermentationen wird in bezug auf flüssige Fettsäuren analysiert.

Die Resultate werden in der nachstehenden Tabelle festgehalten:

Tabelle 8
Effekt von verschiedenen Ionophoren an der Bildung von Gas und flüssigen Fettsäuren bei in-vitro-Pansenfermentation

| Testverbindung | Konzentration der Testverbindung (ppm) | Prozent Erzeugung im Verhältnis zu Kontrollfermentationen: Total flüchtige Fettsäuren | Gaserzeugung | Molverhältnis von Propionat ($C_3$) zu Acetat, + n-Butyrat ($C_2$ + $C_4$)* |
|---|---|---|---|---|
| Monensin B | 10 | 157,8 | 93 | 129 |
| Phenäthylurethan (hergestellt durch Fermentation) | 50 | 149,1 | 92 | 146 |
| Monensin A | 10 | 110,7 | 85 | 162 |
| | 50 | 105,3 | 89 | 165 |
| Narasin | 10 | 102,5 | 88 | 166 |
| | 50 | 106,0 | 86 | 167 |
| Monensin A, Phenäthylurethan (hergestellt durch Fermentation) | 50 | 84,3 | 101,7 | 187 |
| Mono-(4-Bromphenylurethan) von Monensin A | 50 | 89,9 | 103,0 | 165 |
| Monensin B, 1(R)-Phenyläthylurethan | 50 | 82,7 | 103,3 | 167 |
| Kontrolle | 0 | 100 | 100 | 100 |

* Das Verhältnis $C_3$: ($C_2$ + $C_4$) wird auf einen Wert von 100 für unbehandelte Kontrolle normiert.

Verbindungen der Formel I, worin $R^2$ Aryl, Halogenaryl oder Nitroaryl darstellt, und ihre pharmazeutisch verträglichen Salze sind oral wirksam als Antimalariamittel. Diese Wirksamkeit wird durch die folgenden Aktivitäten gegen den Malariaerreger Plasmodium berghei an Mäusen veranschaulicht:

| Verbindung | $ED_{50}$ mg/kg p.o. |
|---|---|
| Monensin A, 4-Chlorphenylurethan | 2,3 |
| Monensin A, Phenylurethan | 4 |
| Monensin A, 4-Bromphenylurethan | 15–20 |

Die Verbindungen der Formel I und ihre pharmazeutisch verträglichen Salze sind oral wirksam als Blutdrucksenkende Mittel. Diese Wirksamkeit wird durch die nachstehende Tabelle 9 veranschaulicht:

Tabelle 9

| Verbindung | orale Dosierung (mg/kg) | Tag mit maximaler Veränderung (%) Blutdruck | | Herzfrequenz | | Durchschnittlicher Blutdruck (mm Hg) vor der | zur Zeit des |
|---|---|---|---|---|---|---|---|
| | | Tag | Veränderung | Tag | Veränderung | Behandlung | maximalen Effekts |
| Monensin B, Phenäthylurethan | 10 | 2 | 13 | 3 | 14 | 216 | 189 |
| Monensin A, Phenäthylurethan | 10 | 5 | 15 | 2 | 18 | 223 | 189 |
| Monensin A, 4-bromphenylurethan* | 1 | 2 | 21 | – | –* | 225 | 178 |
| Monensin A, Phenylurethan* | 10 | 2 | 26 | – | –* | 229 | 170 |
| Monensin A, 4-Chlorphenylurethan | 1 | 5 | 20 | 5 | 19 | 224 | 179 |
| Monensin A, 4-Fluorphenylurethan* | 3 | 2 | 17 | – | –* | 220 | 182 |
| Monensin A, 4-Methylphenylurethan | 1 | 3 | 16 | 4 | 15 | 217 | 182 |

* Verbindungen, welche den Blutdruck, jedoch nicht die Herzfrequenz beeinflussen, sind bevorzugt.

Die erfindungsgemässen Verbindungen können hergestellt werden durch Umsetzung von Monensin A oder B, d.h. eine Verbindung der allgemeinen Formel

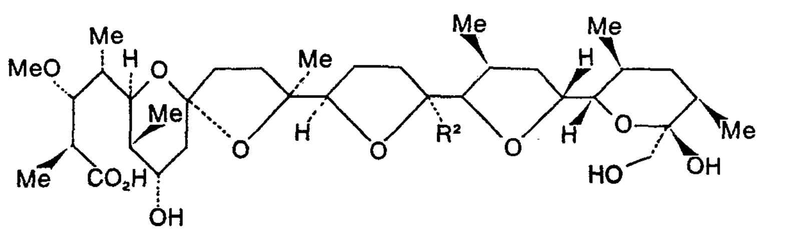

II

worin

$R^2$ Methyl, (Monensin B) oder Äthyl (Monensin A) darstellt,

oder ein Salz davon mit einem Isocyanat der Formel

$$R^1\text{–NCO} \qquad III$$

worin

$R^1$ die oben gegebene Bedeutung hat.

Bevorzugt wird ein Salz der Ausgangsverbindung der Formel II verwendet, insbesondere das Natriumsalz. Das Isocyanat der Formel III wird vorzugsweise in leichtem, beispielsweise etwa 10%igem Überschuss zugegeben, um die Bildung von Monoderivat zu optimieren. Die Umsetzung wird vorzugsweise in einem inerten Lösungsmittel durchgeführt, wie beispielsweise einem chlorierten Kohlenwasserstoff, z.B. Tetrachlorkohlenstoff, Methylenchlorid oder Chloroform; Diäthyläther, Äthylacetat, oder in einem aromatischen Kohlenwasserstoff-Lösungsmittel, wie Benzol oder Toluol. Die Reaktionstemperatur ist nicht kritisch, sie liegt jedoch zwischen etwa 0 °C und dem Siedepunkt des Reaktionsgemisches, vorzugsweise bei etwa Zimmertemperatur.

Beispiel 1

Schüttelkolbengärung mit Streptomyces X-14667

Der Mikroorganismus X-14667 wird auf einer Stärke-Agar-Schrägkultur folgender Zusammen-

setzung (g/l in destilliertem Wasser) gezüchtet und aufbewahrt:

| | |
|---|---|
| Stärke | 10,0 |
| N–Z Amin A | 2,0 |
| Fleischextrakt | 1,0 |
| Hefeextrakt | 1,0 |
| $CoCl_2 \cdot 6H_2O$ | 0,02 |
| Agar | 10,0 |

Die Schrägkultur wird mit dem Mikroorganismus X-14667 beimpft und bei 28 °C 7 bis 14 Tage bebrütet. Ein Teil des Agars mit der sporulierten Kultur wird zur Beimpfung eines 500-ml-Erlenmeyerkolbens mit 100 ml sterilisiertem Impfsubstrat folgender Zusammensetzung (g/l in destilliertem Wasser) verwendet:

| | |
|---|---|
| Tomatenmark | 5,0 |
| getrocknete Rückstände aus der Brennerei | 5,0 |
| Pepton | 5,0 |
| entbitterte Hefe | 5,0 |
| Maisstärke | 20,0 |
| $CaCO_3$ | 1,0 |
| $K_2HPO_4$ | 1,0 |

Der pH-Wert wird vor der Sterilisierung mit wässriger Natriumhydroxidlösung auf 7 eingestellt.

Die beimpfte Nährlösung wird bei 28 °C 48 bis 72 Stunden auf einer Schüttelmaschine (250 Upm; 5 cm Ausschlag) bebrütet.

Eine 3-ml-Portion (3%, V/V) der erhaltenen Kultur wird zur Beimpfung eines 500-ml-Erlenmeyerkolbens mit 100 ml sterilisiertem Nährmedium folgender Zusammensetzung (g/l in destilliertem Wasser) verwendet:

| | |
|---|---|
| Tomatenmark | 5,0 |
| getrocknete Rückstände aus der Brennerei | 5,0 |
| Pepton | 5,0 |
| entbitterte Hefe | 5,0 |
| Maisstärke | 20,0 |
| $CaCO_3$ | 1,0 |
| $K_2HPO_4$ | 1,0 |

Der pH-Wert wird vor dem Autoklavieren mit wässriger Natriumhydroxidlösung auf 7,0 eingestellt.

Die beimpfte Nährlösung wird bei 28 °C 5 Tage auf einer Schüttelmaschine (250 Upm; 5 cm Ausschlag) bebrütet.

Beispiel 2

Tankgärung mit Streptomyces X-14667

Der Mikroorganismus X-14667 wird auf einer Stärke-Casein-Agar-Schrägkultur folgender Zusammensetzung (g/l) gezüchtet und aufbewahrt.

| | |
|---|---|
| Lösliche Stärke | 5,0 |
| Casein | 1,0 |
| $K_2HPO_4$ | 0,5 |
| $MgSO_4$ (wasserfrei) | 0,5 |
| Agar | 20,0 |

Der pH-Wert wird vor dem Autoklavieren mit wässriger Natriumhydroxidlösung auf 7,4 eingestellt.

Die Schrägkultur wird mit dem Mikroorganismus X-14667 beimpft und bei 28 °C 7 bis 10 Tage bebrütet. Ein Teil des Agars mit der sporulierten Kultur wird zur Beimpfung eines 500-ml-Erlenmeyerkolbens mit 100 ml Impfsubstrat folgender Zusammensetzung (g/l in destilliertem Wasser) verwendet:

| | |
|---|---|
| Tomatenmark | 5,0 |
| getrocknete Rückstände aus der Brennerei | 5,0 |
| Pepton | 5,0 |
| entbitterte Hefe | 5,0 |
| Maisstärke | 20,0 |
| $CaCO_3$ | 1,0 |
| $K_2HPO_4$ | 1,0 |

Der pH-Wert wird vor dem Autoklavieren auf 7,0 eingestellt.

Die beimpfte Nährlösung wird 96 Stunden bei 28 °C auf einer Schüttelmaschine (250 Upm; 5 cm Ausschlag) bebrütet.

20 ml (1% V/V) dieser Kultur wird zur Beimpfung eines 6-Liter-Erlenmeyerkolbens mit 2 Liter Nährlösung folgender Zusammensetzung (g/l in destilliertem Wasser) verwendet:

| | |
|---|---|
| Tomatenmark | 5,0 |
| getrocknete Rückstände aus der Brennerei | 5,0 |
| Pepton | 5,0 |
| entbitterte Hefe | 5,0 |
| Maisstärke | 20,0 |
| $CaCO_3$ | 1,0 |
| $K_2HPO_4$ | 1,0 |

Der pH-Wert wird auf 7,0 eingestellt; anschliessend wird 45 Minuten bei 1 bis 1½ kg/cm² autoklaviert.

Die beimpfte Nährlösung wird bei 28 °C 72 Stunden auf einer Schüttelmaschine (250 Upm; 5 cm Ausschlag) bebrütet.

Eine 6-Liter-Portion der erhaltenen Kultur wird zur Beimpfung von 230 Liter Nährmedium folgender Zusammensetzung (g/l in destilliertem Wasser) in einem 380-Liter-Fermenter verwendet:

| | |
|---|---|
| Tomatenmark | 5,0 |
| getrocknete Rückstände aus der Brennerei | 5,0 |
| Pepton | 5,0 |
| entbitterte Hefe | 5,0 |
| Maisstärke | 20,0 |
| $CaCO_3$ | 1,0 |
| $K_2HPO_4$ | 1,0 |
| Schaumbekämpfungsmittel | 0,1 |

Der pH-Wert wird mit wässriger Natriumhydroxidlösung auf 7,0 eingestellt; anschliessend wird 1¼ Stunden mit Dampf von 4 kg/cm² sterilisiert.

Das beimpfte Medium wird mit Druckluft von einer Geschwindigkeit von 9 Liter pro Minute belüftet und mit einem Rührwerk (280 Upm) gerührt. Die Gärung wird bei 28 °C 5 Tage durchgeführt.

Beispiel 3

Isolierung von Monensin A, Monensin B und Diphenäthylharnstoff

Die gesamte Brühe aus einer 230-Liter-Gärung von Streptomyces sp. X-14667 wird bei pH 7,6 mit dem gleichen Volumen Äthylacetat extrahiert. Die Lösungsmittelschicht wird abgetrennt und unter vermindertem Druck auf 1,0 Liter konzentriert.

Das Lösungsmittelkonzentrat wird nacheinander mit gleichen Volumen von 1n wässriger Salzsäure, Wasser, gesättigter wässriger Natriumcarbonatlösung und Wasser gewaschen und anschliessend über Natriumsulfat getrocknet.

Nach weiterer Konzentrierung und Filtration erhält man eine kristalline Mischung, welche Monensin A, Monensin B, Diphenylharnstoff sowie ein Öl enthält.

Beispiel 4
Isolierung von Monensin A und Diphenäthylharnstoff

Die nach Beispiel 3 erhaltenen Mischkristalle werden in 100 ml Methylenchlorid gelöst und mit 1n wässriger Salzsäure gewaschen. Die Lösungsmittelphase wird auf ein kleines Volumen eingedampft. Nach Zugabe von n-Hexan wird Diphenäthylharnstoff (sp. 136 °C) durch Filtration abgetrennt.

Das Filtrat (Mutterlauge) wird mit Diäthyläther verdünnt und mit gesättigter, wässriger Natriumcarbonatlösung gewaschen. Nach dem Konzentrieren und der Zugabe von n-Hexan wird kristallines Monensin B (Fp. 276 °C) abgetrennt; eine zweite Kristallernte wird ebenfalls abgetrennt.

Durch wiederholte Dünnschichtchromatographie an Kieselgel (mit Äthylacetat als Lösungsmittel) wird festgestellt, dass die zweite Kristallernte eine Mischung von Monensin B und Monensin A darstellt.

Beispiel 5
Isolierung von Monensin A Phenäthylurethan und Monensin B Phenäthylurethan

Das Öl aus der ersten Filtration (Beispiel 3) wird in n-Hexan (0,47 Liter) gelöst und 2mal mit 0,47 Liter Acetonitril extrahiert. Die Extrakte werden vereinigt, zu einem Öl konzentriert und mit n-Hexan gewaschen. Der in n-Hexan unlösliche, ölige Feststoff wird in Diäthyläther gelöst, filtriert und in 500 g Kieselgel chromatographiert, wobei als Eluierungsmittel ein Gradient zwischen 5 Liter Methylenchlorid und 5 Liter Methylenchlorid-äthanol (9 : 1) verwendet wird. Es werden Fraktionen zu je etwa 18 ml gesammelt; die Fraktionen 120 bis 180 werden zur wiederholten Chromatographie vereinigt. Die Fraktionen 185 bis 235 werden vereinigt, unter vermindertem Druck zu einem öligen Feststoff konzentriert, in Diäthyläther gelöst und nacheinander mit 1n wässriger Salzsäure, gesättigter wässriger Natriumcarbonatlösung und Wasser gewaschen. Das Lösungsmittel wird unter vermindertem Druck eingedampft; man erhält das Natriumsalz von Monensin B Phenäthylurethan als ein Pulver, Fp. 70 °C; $[\alpha]_D$ +48° ($CHCl_3$, C = 1%), +44° ($CH_3OH$, C = 1%).

Elementaranalyse: Ber.: $C_{44}H_{68}NO_{12}Na$ (826.98): C, 63,90; H, 8,41; N, 1,69; Na, 2,78. Gef. C, 64,45; H, 8,68; N, 1,76; Na, 2,31.

200 ml des Natriumsalzes von Monensin B Phenäthylurethan werden in Äthylacetat gelöst und mit 1n wässriger Salzsäure gewaschen. Das Lösungsmittel wird unter vermindertem Druck eingedampft. Man erhält 100 g Monensin B Phenäthylurethan als ein beigefarbenes Pulver, Fp. 50 °C $[\alpha]_D$ + 59,6° ($CHCl_3$, C = 1%); + 45,8° ($CH_3OH$, C = 1%). Elementaranalyse: Ber.: $C_{44}H_{69}NO_{12}$ (804,4): C, 65,65; H, 8,76; N, 1,74; O, 23,85. Gef.: C, 65,53; H, 8,16; N, 1,49; O, 24,16.

Die vereinigten Fraktionen 120 bis 180 werden unter vermindertem Druck zu einem Öl konzentriert und an einer 700 g Kieselgelkolonne umchromatographiert. Die Kolonne wird anfänglich mit 4 Liter Äthylacetat eluiert, anschliessend mit 4 Liter Äthylacetat/Aceton (1 : 1) und schliesslich mit 4 Liter Äthylacetat/Äthanol (95 : 1). Die vereinigten Fraktionen 29 bis 35 liefern nach dem Konzentrieren Diphenylharnstoff. Die vereinigten Fraktionen 86 bis 121 liefern nach dem Abdampfen des Lösungsmittels 2 g Monensin B Phenäthylurethan. Die vereinigten Fraktionen 55 bis 85 werden unter vermindertem Druck zu einem öligen Feststoff konzentriert und mit Hilfe von n-Hexan/Aceton (8 : 2) umchromatographiert. Die Zweikomponenten-Mischung wird in Monensin B Phenäthylurethan und Monensin A 2-Phenyläthylurethan aufgetrennt, letzteres als ein Pulver nach dem Eindampfen des Lösungsmittels, Fp. 103 °C $[\alpha]_D$ + 50° ($CHCl_3$, C = 1%). Elementaranalyse: Ber.: $C_{45}H_{70}NO_{12}Na$ (840,04); C, 64,34; H, 8,40; N, 1,67; Na, 2,73. Gef. C, 64,43; H, 8,29; N, 1,92; Na, 2,49.

Die oben angegebenen Strukturen der Endprodukte werden durch Synthese aus Monensin A bzw. B bestätigt. Das halbsynthetische Material wird in beiden Fällen hergestellt durch Auflösung von 1 mMol des Antibiotikums in Benzol und Zugabe von 1,2 mMol Phenäthylisocyanat. Nach 12stündiger Reaktion werden die Endprodukte durch Waschen des Reaktionsgemisches mit wässriger Natriumcarbonatlösung und Abdampfen des Lösungsmittels unter vermindertem Druck aufgearbeitet. Die erhaltenen rohen Endprodukte werden durch Chromatographie an Kieselgel, wie für die Fermentations-Endprodukte beschrieben, gereinigt. Die halbsynthetische und durch Fermentation erhaltenen Produkte zeigten sich durch Schmelzpunkt, $[\alpha]_D$, IR, NMR und MS als identisch.

Das in der obigen Reaktion verwendete Phenäthylisocyanat kann wie folgt hergestellt werden:

40 g Phenäthylamin in 300 ml trockenem, mit trockenem Chlorwasserstoffgas gesättigtem Toluol werden bis zum Auftreten eines schweren weissen Niederschlages gemischt. Zusätzlich werden 200 ml trockenes Toluol mit 50 ml Phosgen (12,5% in Benzol) hinzugefügt. Das Gemisch wird 10 Minuten unter Rückflussbedingungen erhitzt; anschliessend werden 450 ml Phosgen langsam innerhalb 30 Minuten hinzugefügt und das erhaltene Gemisch 4 Stunden unter Rückflussbedingungen erhitzt. Das Reaktionsgemisch wird langsam auf Zimmertemperatur gekühlt und das Lösungsmittel unter vermindertem Druck entfernt, wonach ein gelbes Öl erhalten wird. Nach Destillation dieses Öls bei 82 bis 83 °C/0,1 mm erhält man 1,8 g Phenäthylisocyanat.

Beispiel 6

Herstellung des 4-Bromphenylurethans von Monensin A aus Monensin A (freie Säure)

10 mMol (6,89 g) Monensin A-Hydrat werden in 100 ml Benzol gelöst. Diese Lösung wird mit einem Überschuss (11 mMol) 4-Bromphenylisocyanat im gleichen Volumen Benzol versetzt und das Reaktionsgemisch bei Zimmertemperatur gerührt. Die Reaktion wird durch Dünnschichtchromatographie an Kieselgel mit Äthylacetat-n-Hexan-Äthanol (80 : 20 : 2) als Lösungsmittel und Vanillin-Phosphorsäure als Entwickler verfolgt.

Nach einer Woche wird das Reaktionsgemisch nacheinander mit 0,5n wässriger Salzsäure, Wasser, gesättigter wässriger Natriumcarbonatlösung und Wasser gewaschen. Die Benzollösung wird über Natriumsulfat getrocknet und unter vermindertem Druck zu einem amorphen Feststoff eingedampft. Nach dem Kristallisieren aus Acetonitril erhält man das Natriumsalz des 4-Bromphenylurethans von Monensin A, Fp. 201 bis 203 °C $[\alpha]_D$ +65,3° ($CHCl_3$, C = 1%).

Elementaranalyse: Ber.: für $C_{43}H_{66}O_{12}NBrNa$ (891,87) % C, 57,90; H, 7,46; N, 1,57; Br, 8,96. Gef. % C, 58,57; H, 7,36; N, 1,23; Br. 8,90.

Beispiel 7

Herstellung des 4-Bromphenylurethans von Monensin A aus dem Natriumsalz von Monensin

Ausgehend von 5 g Natriumsalz von Monensin A (7 mMol) und 2 g 4-Bromphenylisocyanat lässt man die Reaktion wie gemäss Beispiel 6 laufen, jedoch scheint das Natriumsalz schneller zu reagieren. Gemäss Dünnschichtchromatogramm scheint die Reaktion nach 2 Tagen vollendet zu sein.

Nach dem Aufarbeiten in oben angegebener Weise erhält man das 4-Bromphenylurethan von Monensin A.

Beispiel 8

In der gleichen Weise wie in den Beispielen 6 und 7 angegeben erhält man durch Verwendung des entsprechenden Isocyanats folgende Verbindungen:

| Verbindung | Schmelzpunkt °C | optische Drehung $\alpha^{D,o}$ |
|---|---|---|
| Monensin A, Methylurethan | 191–193 | |
| Monensin A, 4-Bromphenäthylurethan | 201–203 | |
| Monensin B, (R)-1-Phenyläthylurethan | 89–93 | |
| Monensin A, Phenylurethan | 199–210 | |
| Monensin A, 4-Chlorphenylurethan | 199–207 | |
| Monensin B, (S)-1-Phenyläthylurethan | 116–120 | |
| Monensin A, Cyclohexylurethan | 110–123 | |
| Monensin A, 2-(Phenyl)-cyclopropylurethan | 119–124 | |
| Monensin A, 4-Fluorphenylurethan | 199–223 | |
| Monensin A, 4-Nitrophenylurethan | 189–192 | + 104,7 (1%, $CH_3CN$) |
| Monensin A, 4-Methylphenylurethan | 220 | + 59,6 (1%, $CHCl_3$) |
| Monensin A, 4-Jodphenylurethan | 203–206 | + 67,6 (1%, $CHCl_3$) |
| Monensin A, n-Butylurethan | | + 59,2 (1%, $CHCl_3$) |
| Monensin A, 4-Phenoxyphenylurethan | 210–213 | + 67,9 (1%, $CHCl_3$) |

Beispiel 9

Herstellung von Tabletten (Nassgranulierung)

| Bestandteile | mg/Tablette | mg/Tablette | mg/Tablette |
|---|---|---|---|
| 1. Monensin B Phenäthylurethan oder Monensin A Phenäthylurethan | 15 | 30 | 60 |
| 2. Milchzucker | 188 | 173 | 188 |
| 3. modifizierte Stärke | 25 | 25 | 30 |
| 4. vorgelatinierte Stärke | 20 | 20 | 20 |
| 5. destilliertes Wasser q.s. | – | – | – |
| 6. Magnesiumstearat | 2 | 2 | 2 |
| Gewicht einer Tablette | 250 mg | 250 mg | 300 mg |

PROZEDERE:

1) No. 1–4 werden in einem geeigneten Mixer gemischt.

2) Das erhaltene Gemisch wird mit genügend destilliertem Wasser granuliert und anschliessend gemahlen.

3) Das Mahlgut wird in einem Ofen getrocknet.

4) Das getrocknete Produkt wird gemahlen und mit Magnesiumstearat vermischt.

5) Das erhaltene Produkt wird 3 Minuten in einer Tablettierungsmaschine komprimiert.

Beispiel 10 Tabletten (Direkte Kompression)

| Bestandteile | mg/Tablette | mg/Tablette | mg/Tablette |
|---|---|---|---|
| 1. Monensin B Phenäthylurethan oder Monensin A Phenäthylurethan | 15 | 30 | 60 |
| 2. Milchzucker | 207 | 192 | 162 |
| 3. Avicel | 45 | 45 | 45 |
| 4. Stärke für direkte Kompression | 30 | 30 | 30 |
| 5. Magnesiumstearat | 3 | 3 | 3 |
| Gewicht einer Tablette | 300 mg | 300 mg | 300 mg |

PROZEDERE:

1) No. 1 wird mit der gleichen Menge Milchzukker gut vermischt.

2) Diese Mischung wird mit No. 3, 4 und dem restlichen Milchzucker gut vermischt.

3) Das Magnesiumstearat wird hinzugefügt und das Ganze 3 Minuten gemischt.

4) Das erhaltene Produkt wird an einer Tablettierungsmaschine komprimiert.

Beispiel 11 Kapseln

| Bestandteile | mg/Kapsel | mg/Kapsel | mg/Kapsel |
|---|---|---|---|
| 1. Monensin B Phenäthylurethan oder Monensin A Phenäthylurethan | 15 | 30 | 60 |
| 2. Milchzucker | 239 | 224 | 194 |
| 3. Stärke | 30 | 30 | 30 |
| 4. Talk | 15 | 15 | 15 |
| 5. Magnesiumstearat | 1 | 1 | 1 |
| Kapselfüllgewicht | 300 mg | 300 mg | 300 mg |

PROZEDERE:

1) No. 1–3 werden in einem geeigneten Mixer gemischt.

2) Talk und Magnesiumstearat werden hinzugefügt und das Ganze kurz gemischt.

3) Das erhaltene Produkt wird in einer entsprechenden Maschine in Kapseln abgefüllt.

Beispiel 12

Ein Tierfutter zur Verwendung gemäss der Erfindung hat z.B. folgende Zusammensetzung:

| | Gewichtsprozent | Gewichtsprozent auf Basis der Trockensubstanz |
|---|---|---|
| Maissilofutter | 21,6 | 10,7 |
| Gewalzter Trockenmais | 70,7 | 80,0 |
| Proteinzusatz | 7,7 | 9,3 |

Proteinzusatz

| | |
|---|---|
| Alfalfa Heu | 15,49 |
| Gewalztes Trockenkorn von Sorghum | 3,72 |
| Baumwollsamenmehl | 63,56 |
| Kalkstein | 8,97 |
| Salz (NaCl) | 3,40 |
| Spurenmineralien | 0,11 |
| Vitamin A Vormischung | 0,13 |
| KCl | 4,36 |
| Monensin A Phenäthylurethan Vormischung (15 Gewichtsprozent) | 0,26 |
| | 100,0 |

Monensin A Phenäthylurethan Vormischung

| | |
|---|---|
| Monensin A Phenäthylurethan | 15 |
| Reissamenhülsen | 85 |

**Patentansprüche für die Vertragsstaaten: BE, CH, DE, FR, IT, LU, NL, SE**

1. Urethan-Derivate der allgemeinen Formel

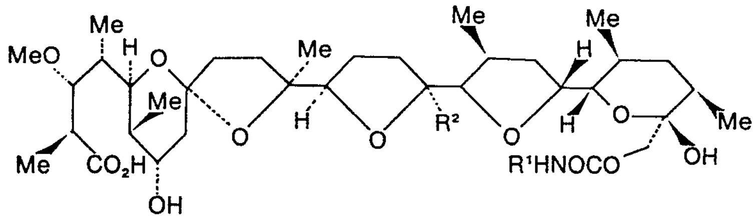

I

in der

$R^1$ Phenyl, $C_{1-7}$-Alkyl-Phenyl, Halogen-Phenyl, Nitro-Phenyl, Phenoxy-Phenyl, $C_{1-7}$-Alkyl, $C_{3-7}$-Cycloalkyl, Phenyl-$C_{1-7}$-Alkyl, $C_{1-7}$-Alkanoyl-Phenyl, $C_{1-7}$-Alkoxy-Phenyl oder Phenyl-$C_{3-7}$-Cycloalkyl und

$R^2$ Methyl oder Äthyl darstellen, und ihre pharmazeutisch verträglichen Salze.

2. Eine Verbindung gemäss Anspruch 1, worin $R^1$ Phenyl, $C_{1-7}$-Alkyl-Phenyl, Halogen-Phenyl, Nitro-Phenyl, $C_{1-7}$-Alkoxy-Phenyl, Phenoxy-Phenyl, $C_{1-7}$-Alkanoyl-Phenyl oder $C_{3-7}$-Cycloalkyl darstellt.

3. Eine Verbindung gemäss Anspruch 1, worin $R^1$ Phenyl, $C_{1-7}$-Alkyl-Phenyl oder Halogen-Phenyl darstellt.

4. Eine Verbindung gemäss einem der Ansprüche 1–3, worin $R^2$ Äthyl darstellt.

5. Eine Verbindung gemäss einem der Ansprüche 1–4, worin $R^1$ Phenyl oder Halogen-Phenyl darstellt.

6. Eine Verbindung gemäss Anspruch 5, worin $R^1$ Phenyl und $R^2$ Äthyl darstellen.

7. Eine Verbindung gemäss Anspruch 5, worin $R^1$ 4-Chlorphenyl und $R^2$ Äthyl darstellen.

8. Eine Verbindung gemäss Anspruch 5, worin $R^1$ 4-Bromphenyl und $R^2$ Äthyl darstellen.

9. Eine Verbindung gemäss Anspruch 5, worin $R^1$ 4-Fluorphenyl und $R^2$ Äthyl darstellen.

10. Eine Verbindung gemäss Anspruch 5, worin $R^1$ 4-Nitrophenyl und $R^2$ Äthyl darstellen.

11. Eine Verbindung gemäss Anspruch 1 oder 4, worin $R^1$ Phenäthyl darstellt.

12. Verbindungen gemäss einem der Ansprüche 1–11, als pharmazeutische Wirkstoffe.

13. Verbindungen gemäss einem der Ansprüche 1–11, als pharmazeutische Wirkstoffe zur Behandlung und Prophylaxe von bakteriellen Krankheiten, Coccidiose, erhöhtem Blutdruck, Malaria und/oder Schweinedysenterie.

14. Verbindungen gemäss einem der Ansprüche 1–11, als Mittel zur Erhöhung des Futternutzeffektes bei Wiederkäuern und bei monogastrischen Tieren, die faseriges Pflanzenmaterial im Blinddarm vergären.

15. Verfahren zur Herstellung der Urethan-Derivate gemäss einem der Ansprüche 1–11, dadurch gekennzeichnet, dass man eine Verbindung der Formel

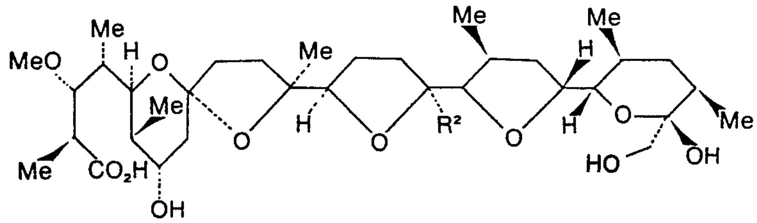

II

in der

$R^2$ Methyl oder Äthyl darstellt, mit einem Isocyanat der allgemeinen Formel

$R^1$–NCO III

in der

$R^1$ die in Anspruch 1 gegebene Bedeutung hat, umsetzt.

16. Verfahren nach Anspruch 15, dadurch gekennzeichnet, dass man das Isocyanat der Formel III in leichtem, beispielsweise etwa 10%igem Überschuss zugibt.

17. Verfahren zur Herstellung der Verbindungen gemäss Anspruch 11, d.h. worin $R^1$ Phenäthyl darstellt, dadurch gekennzeichnet, dass man eine Subkultur einer der Stämme Streptomyces sp. X-14667 NRRL 11336 (ATCC 31551), Streptomyces sp. X-14573 NRRL 11337 (ATCC 31552) und Streptomyces sp. X-14575 NRRL 11338 (ATCC 31553) in einer Kohlenhydrate und stickstoffhaltige Nährstoffe enthaltenden wässrigen Lösung unter submersen aeroben Bedingungen kultiviert und anschliessend das Endprodukt aus der Lösung isoliert.

18. Pharmazeutische Präparate, gekennzeichnet durch einen Gehalt an einer Verbindung gemäss einem der Ansprüche 1–11.

19. Pharmazeutische Präparate zur Behandlung und Prophylaxe von bakteriellen Krankheiten, Coccidiose, erhöhtem Blutdruck, Malaria und/oder Schweinedysenterie, gekennzeichnet durch einen Gehalt an einer Verbindung gemäss einem der Ansprüche 1–11.

20. Vormischung oder Komposition zum unmittelbaren Verbrauch und zur Erhöhung des Futternutzeffektes bei Wiederkäuern und bei monogastrischen Tieren, die faseriges Pflanzenmaterial im Blinddarm vergären, gekennzeichnet durch einen Gehalt an einer Verbindung gemäss einem der Ansprüche 1–11.

**Patentansprüche für den Vertragsstaat: AT**

1. Verfahren zur Herstellung von Urethan-Derivaten der allgemeinen Formel

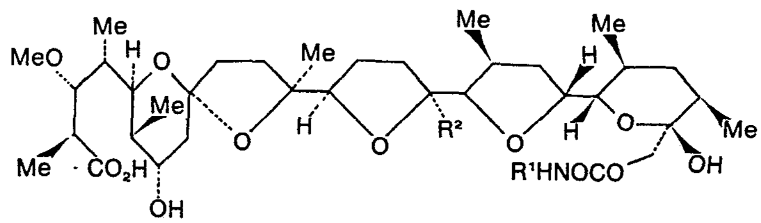

I

in der

$R^1$ Phenyl, $C_{1-7}$-Alkyl-Phenyl, Halogen-Phenyl, Nitro-Phenyl, Phenoxy-Phenyl, $C_{1-7}$-Alkyl, $C_{3-7}$-Cycloalkyl, Phenyl-$C_{1-7}$-Alkyl, $C_{1-7}$-Alkanoyl-Phenyl, $C_{1-7}$-Alkoxy-Phenyl oder Phenyl-$C_{3-7}$-Cycloalkyl und

$R^2$ Methyl oder Äthyl darstellen, und ihrer pharmazeutisch verträglichen Salze, dadurch gekennzeichnet, dass man eine Verbindung der Formel

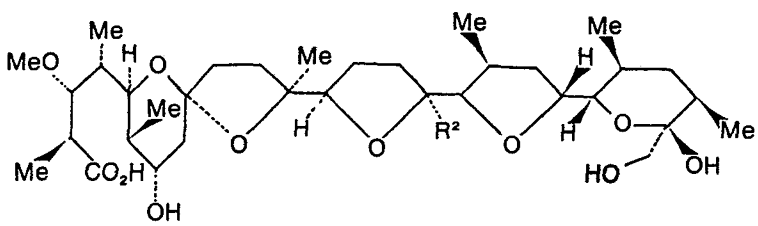

II

in der

$R^2$ Methyl oder Äthyl darstellt, mit einem Isocyanat der Formel

$R^1$–NCO III

in der

$R^1$ die oben gegebene Bedeutung hat, umsetzt.

2. Verfahren nach Anspruch 1 zur Herstellung von Verbindungen, worin $R^1$ Phenyl, $C_{1-7}$-Alkyl-Phenyl, Halogen-Phenyl, Nitro-Phenyl, $C_{1-7}$-Alkoxy-Phenyl, Phenoxy-Phenyl, $C_{1-7}$-Alkanoyloxy-Phenyl oder $C_{3-7}$-Cycloalkyl darstellt, dadurch gekennzeichnet, dass man ein entsprechend substituiertes Isocyanat der Formel III verwendet.

3. Verfahren nach Anspruch 1 zur Herstellung von Verbindungen, worin $R^1$ Phenyl, $C_{1-7}$-Alkyl-Phenyl oder Halogen-Phenyl darstellt, dadurch gekennzeichnet, dass man ein entsprechend substituiertes Isocyanat der Formel III verwendet.

4. Verfahren nach einem der Ansprüche 1–3 zur Herstellung von Verbindungen, worin $R^2$ Äthyl darstellt, dadurch gekennzeichnet, dass man eine entsprechend substituierte Ausgangsverbindung der Formel II einsetzt.

5. Verfahren nach einem der Ansprüche 1–4 zur Herstellung von Verbindungen, worin $R^1$ Phenyl oder Halogen-Phenyl darstellt, dadurch gekennzeichnet, dass man ein entsprechend substituiertes Isocyanat der Formel III einsetzt.

6. Verfahren nach Anspruch 5 zur Herstellung von Verbindungen, worin $R^1$ Phenyl und $R^2$ Äthyl darstellen, dadurch gekennzeichnet, dass man entsprechend substituierte Ausgangsverbindungen der Formel II und III einsetzt.

7. Verfahren nach Anspruch 5 zur Herstellung von Verbindungen, worin $R^1$ 4-Chlorphenyl und $R^2$ Äthyl darstellen, dadurch gekennzeichnet, dass man entsprechend substituierte Ausgangsverbindungen der Formeln II und III einsetzt.

8. Verfahren nach Anspruch 5 zur Herstellung von Verbindungen, worin $R^1$ 4-Bromphenyl und $R^2$ Äthyl darstellen, dadurch gekennzeichnet, dass man entsprechend substituierte Ausgangsverbindungen der Formeln II und III einsetzt.

9. Verfahren nach Anspruch 5 zur Herstellung von Verbindungen, worin $R^1$ 4-Fluorphenyl und $R^2$ Äthyl darstellen, dadurch gekennzeichnet, dass man entsprechend substituierte Ausgangsverbindungen der Formeln II und III einsetzt.

10. Verfahren nach Anspruch 5 zur Herstellung von Verbindungen, worin $R^1$ 4-Nitrophenyl und $R^2$ Äthyl darstellen, dadurch gekennzeichnet, dass man entsprechend substituierte Ausgangsverbindungen der Formeln II und III einsetzt.

11. Verfahren nach Anspruch 1 oder 4 zur Herstellung von Verbindungen, worin $R^1$ Phenäthyl darstellt, dadurch gekennzeichnet, dass man eine entsprechend substituierte Ausgangsverbindung der Formel II einsetzt.

12. Verfahren nach einem der Ansprüche 1–11, dadurch gekennzeichnet, dass man das Isocyanat der Formel III in leichtem, beispielsweise etwa 10%igem Überschuss zusetzt.

13. Verfahren zur Herstellung von Verbindungen der Formel I nach Anspruch 1 oder 4, worin $R^1$

Phenäthyl darstellt, dadurch gekennzeichnet, dass man eine Subkultur einer der Stämme Streptomyces sp. X-14667 NRRL 11336 (ATCC 31551), Streptomyces sp. X-14573 NRRL 11337 (ATCC 31552) und Streptomyces sp. X-14575 NRRL 11338 (ATCC 31553) in einer Kohlenhydrate und stickstoffhaltige Nährstoffe enthaltenden wässrigen Lösung unter submersen aeroben Bedingungen kultiviert und anschliessend das Endprodukt aus der Lösung isoliert.

**Claims for the contracting States: BE, CH, DE, FR, IT, LU, NL, SE**

1. Urethane derivatives of the general formula

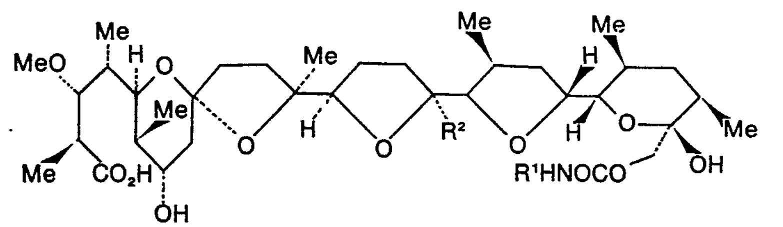

I

wherein

$R^1$ is phenyl, $C_{1-7}$-alkyl-phenyl, halogen-phenyl, nitro-phenyl, phenoxy-phenyl, $C_{1-7}$-alkyl, $C_{3-7}$ cycloalkyl, phenyl-$C_{1-7}$-alkyl, $C_{1-7}$-alkanoyl-phenyl, $C_{1-7}$-alkoxy-phenyl or phenyl-$C_{3-7}$-cycloalkyl and

$R^2$ is methyl or ethyl, and their pharmaceutically acceptable salts.

2. A compound according to claim 1, wherein $R^1$ is phenyl, $C_{1-7}$-alkyl-phenyl, halogen-phenyl, nitrophenyl, $C_{1-7}$-alkoxy-phenyl, phenoxy-phenyl, $C_{1-7}$-alkanoyl-phenyl or $C_{3-7}$-cycloalkyl.

3. A compound according to claim 1, wherein $R^1$ is phenyl, $C_{1-7}$-alkyl-phenyl or halogen-phenyl.

4. A compound according to any one of claims 1–3, wherein $R^2$ is ethyl.

5. A compound according to any one of claims 1–4, wherein $R^1$ is phenyl or halogen-phenyl.

6. A compound according to claim 5, wherein $R^1$ is phenyl and $R^2$ is ethyl.

7. A compound according to claim 5, wherein $R^1$ is 4-chlorophenyl and $R^2$ is ethyl.

8. A compound according to claim 5, wherein $R^1$ is 4-bromophenyl and $R^2$ is ethyl.

9. A compound according to claim 5, wherein $R^1$ is fluorophenyl and $R^2$ is ethyl.

10. A compound according to claim 5, wherein $R^1$ is 4-nitrophenyl and $R^2$ is ethyl.

11. A compound according to claim 1 or 4, wherein $R^1$ is phenethyl.

12. Compounds according to any one of claims 1–11 as pharmaceutical active substances.

13. Compounds according to any one of claims 1–11 as pharmaceutical active substances for treatment and prophylaxis of bacterial diseases, coccidiosis, hypertension, malaria and/or swine dysentery.

14. Compounds according to any one of claims 1–11 for use in increasing the efficiency of feed utilisation in ruminants and monogastric animals which ferment fibrous vegetable matter in the cecum.

15. A process for the manufacture of the urethane derivatives according to any one of claims 1–11, which comprises reacting a compound of the formula

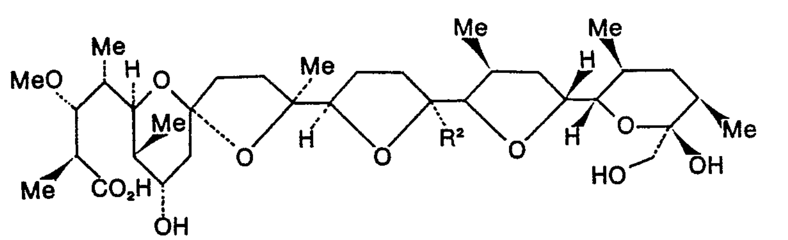

II

wherein

$R^2$ is methyl or ethyl with an isocyanate of the formula

$$R^1\text{–NCO} \qquad III$$

wherein

$R^1$ is as in claim 1.

16. A process according to claim 15, wherein the isocyanate of formula III is added in a slight excess, for example about a 10% excess.

17. A process for the manufacture of the compounds according to claim 11, i.e. where $R^1$ is phenethyl, which comprises cultivating a subculture of one of the strains Streptomyces sp. X-14667 NRRL 11336 (ATCC 31551), Streptomyces sp. X-14573 NRRL 11337 (ATCC 31552) and Streptomyces sp. X-14575 NRRL 11338 (ATCC 31553) in an aqueous carbohydrate solution containing a nitrogenous nutrient under submerged aerobic conditions and thereafter isolating the end product from said solution.

18. Pharmaceutical preparations containing a compound according to any one of claims 1–11.

19. Pharmaceutical preparations for the treatment and prophylaxis of bacterial diseases, coccidiosis, hypertension, malaria and/or swine dysentery containing a compound according to any one of claims 1–11.

20. A premix or a composition for ready consumption which increases the efficiency of feed utilisation in ruminants and monogastric animals which ferment fibrous vegetable matter in the cecum containing a compound according to any one of claims 1–11.

**Claims for contracting state: AT**

1. Process for the manufacture of urethane derivatives of the general formula

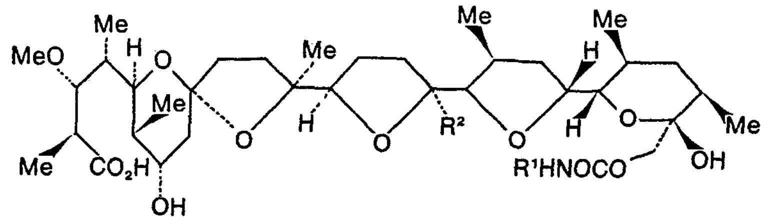

I

wherein

$R^1$ is phenyl, $C_{1-7}$-alkyl-phenyl, halogen-phenyl, nitro-phenyl, phenoxy-phenyl, $C_{1-7}$-alkyl, $C_{3-7}$-cycloalkyl, phenyl-$C_{1-7}$-alkyl, $C_{1-7}$-alkanoyl-phenyl, $C_{1-7}$-alkoxy-phenyl or phenyl-$C_{3-7}$-cycloalkyl and

$R^2$ is methyl or ethyl and their pharmaceutically acceptable salts, which comprises reacting a compound of the formula

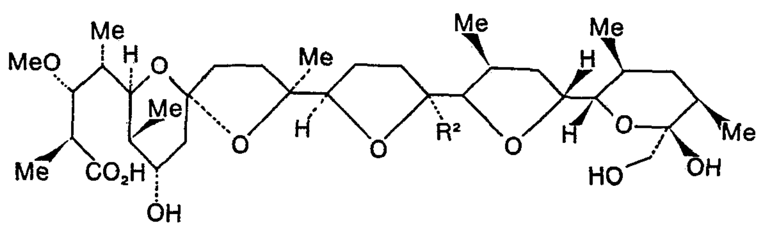

II

wherein

$R^2$ is methyl or ethyl with an isocyanate of the formula

$$R^1\text{-NCO} \qquad III$$

wherein

$R^1$ is as given above.

2. Process according to claim 1 for the manufacture of compounds, wherein $R^1$ is phenyl, $C_{1-7}$-alkyl-phenyl, halogen-phenyl, nitro-phenyl, $C_{1-7}$-alkoxy-phenyl, phenoxy-phenyl, $C_{1-7}$-alkanoyloxy-phenyl or $C_{3-7}$-cycloalkyl, which comprises using a correspondingly substituted isocyanate of the formula III.

3. Process according to claim 1 for the manufacture of compounds, wherein $R^1$ is phenyl, $C_{1-7}$-alkyl-phenyl or halogen-phenyl, which comprises using a correspondingly substituted isocyanate of the formula III.

4. Process according to any one of claims 1–3 for the manufacture of compounds, wherein $R^2$ is ethyl, which comprises using a correspondingly substituted starting compound of the formula II.

5. Process according to any one of claims 1–4 for the manufacture of compounds, wherein $R^1$ is phenyl or halogen-phenyl, which comprises using a correspondingly substituted isocyanate of the formula III.

6. Process according to claims 5 for the manufacture of compounds, wherein $R^1$ is phenyl and $R^2$ is ethyl, which comprises using correspondingly substituted starting compounds of the formulae II and III.

7. Process according to claim 5 for the manufacture of compounds, wherein $R^1$ is 4-chlorophenyl and $R^2$ is ethyl, which comprises using correspondingly substituted starting compounds of the formulae II and III.

8. Process according to claim 5 for the manufacture of compounds, wherein $R^1$ is 4-bromophenyl and $R^2$ is ethyl, which comprises using correspondingly substituted starting compounds of the formulae II and III.

9. Process according to claim 5 for the manufacture of compounds, wherein $R^1$ is 4-fluorophenyl and $R^2$ is ethyl, which comprises using correspondingly substituted starting compounds of the formulae II and III.

10. Process according to claim 5 for the manufacture of compounds, wherein $R^1$ is 4-nitrophenyl and $R^2$ is ethyl, which comprises using correspondingly substituted starting compounds of the formulae II and III.

11. Process according to claims 1 or 4 for the manufacture of compounds, wherein $R^1$ is phenethyl, which comprises using correspondingly substituted starting compounds of the formula II.

12. Process according to any one of claims 1–11, which comprises adding the isocyanate of the formula III in a slight excess, for example about a 10% excess.

13. Process according to claims 1 or 4 for the manufacture of compounds, wherein $R^1$ is phenethyl, which comprises cultivating a subculture of one of the strains Streptomyces sp. X-14667 NRRL 11336 (ATCC 31551), Streptomyces sp. X-14573 NRRL 11337 (ATCC 31552) and Streptomyces sp.

X-14575 NRRL 11338 (ATCC 31553) in an aqueous carbohydrate solution containing a nitrogenous nutrient under submerged aerobic conditions and thereafter isolating the end product from said solution.

**Revendications pour les Etats contractants: BE, CH, DE, FR, IT, LU, NL, SE**

1. Dérivés d'uréthane de formule générale

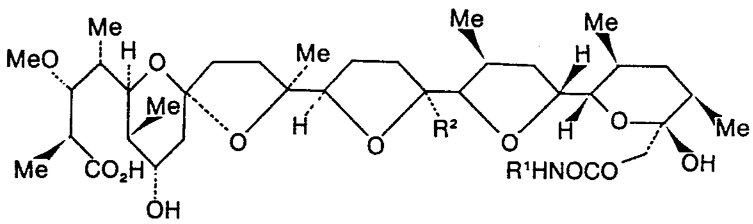

I

où

$R^1$ représente un phényle, un alcoyle en $C_1$ à $C_7$-phényle, un halogénophényle, un nitrophényle, un phénoxy-phényle, un alcoyle en $C_1$ à $C_7$, un cycloalcoyle en $C_3$ à $C_7$, un phényl-alcoyle en $C_1$ à $C_7$, un alcanoyle en $C_1$ à $C_7$-phényle, un alcoxy en $C_1$ à $C_7$-phényle ou un phényl-cycloalcoyle en $C_3$ à $C_7$ et

$R^2$ représente un méthyle ou un éthyle, et leurs sels pharmaceutiquement acceptables.

2. Composé selon la revendication 1, où $R^1$ représente un phényle, un alcoyle en $C_1$ à $C_7$-phényle, un halogénophényle, un nitrophényle, un alcoxy en $C_1$ à $C_7$-phényle, un phénoxyphényle, un alcanoyle en $C_1$ à $C_7$-phényle ou un cycloalcoyle en $C_3$ à $C_7$.

3. Composé selon la revendication 1, où $R^1$ représente un phényle, un alcoyle en $C_1$ à $C_7$-phényle ou un halogénophényle.

4. Composé selon l'une des revendications 1 à 3, où $R^2$ représente un éthyle.

5. Composé selon l'une des revendications 1 à 4, où $R^1$ représente un phényle ou un halogénophényle.

6. Composé selon la revendication 5, où $R^1$ représente un phényle et $R^2$ un éthyle.

7. Composé selon la revendication 5, où $R^1$ représente un 4-chlorophényle et $R^2$ un éthyle.

8. Composé selon la revendication 5, où $R^1$ représente un 4-bromophényle et $R^2$ un éthyle.

9. Composé selon la revendication 5, où $R^1$ représente un 4-fluorophényle et $R^2$ un éthyle.

10. Composé selon la revendication 5, où $R^1$ représente un 4-nitrophényle et $R^2$ un éthyle.

11. Composé selon l'une des revendications 1 ou 4, où $R^1$ représente un phénéthyle.

12. Composés selon l'une des revendications 1–11, comme substances actives pharmaceutiques.

13. Composés selon l'une des revendications 1–11, comme substances actives pharmaceutiques pour le traitement et la prophylaxie des maladies bactériennes, de la coccidiose, de l'hypertension, de la malaria et/ou de la dysenterie du porc.

14. Composés selon l'une des revendications 1–11, comme agents pour accroître l'effet d'utilisation du fourrage chez les ruminants et chez les animaux monogastriques qui fermentent de la matière végétale fibreuse dans le caecum.

15. Procédé de préparation des dérivés d'uréthane selon l'une des revendications 1–11, caractérisé en ce qu'on fait réagir un composé de formule

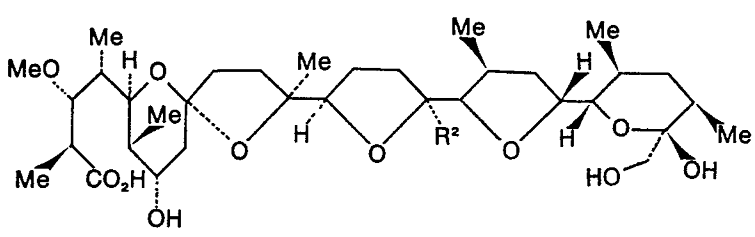

II

où

$R^2$ représente un méthyle ou un éthyle, avec un isocyanate de formule générale

$R^1$–NCO III

où

$R^1$ a la signification donnée dans la revendication 1.

16. Procédé selon la revendication 15, caractérisé en ce qu'on ajoute l'isocyanate de formule III en léger excès, par exemple d'environ 10%.

17. Procédé de préparation des composés selon la revendication 11, c'est-à-dire où $R^1$ représente un phénéthyle, caractérisé en ce qu'on cultive une sous-culture de l'une des souches Streptomyces sp. X-14667 NRRL 11336 (ATCC 31551), Streptomyces sp. X-14573 NRRL 11337 (ATCC 31552) et Streptomyces sp. X-14575 NRRL 11338 (ATCC 31553) dans une solution aqueuse contenant des hydrates de carbone et des matières nutritives azotées dans des conditions aérobies submergées, puis en ce qu'on isole le produit final à partir de la solution.

18. Préparations pharmaceutiques caractérisées en ce qu'elles contiennent un composé selon l'une des revendications 1–11.

19. Préparations pharmaceutiques pour le traitement et la prophylaxie des maladies bactériennes, de la coccidiose, de l'hypertension, de la malaria et/cu de la dysenterie du porc, caractérisées en ce qu'elles contiennent un composé selon l'une des revendications 1–11.

20. Pré-mélange ou composition destinée à l'utilisation immédiate pour accroître l'effet d'utilisation du fourrage chez les ruminants et chez les animaux monogastriques qui fermentent de la matière végétale fibreuse dans le caecum, caractérisés en ce qu'ils contiennent un composé selon l'une des revendications 1–11.

**Revendications pour l'Etat: AT**

1. Procédé de préparation de dérivés d'uréthane de formule générale

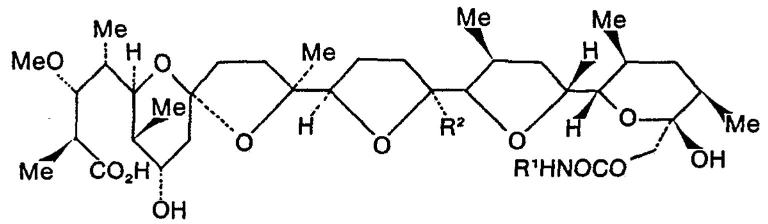

I

où

$R^1$ représente un phényle, un alcoyle en $C_1$ à $C_7$-phényle, un halogénophényle, un nitrophényle, un phénoxyphényle, un alcoyle en $C_1$ à $C_7$, un cycloalcoyle en $C_3$ à $C_7$, un phénylalcoyle en $C_1$ à $C_7$, un alcanoyle en $C_1$ à $C_7$-phényle, un alcoxy en $C_1$ à $C_7$-phényle ou un phénylcycloalcoyle en $C_3$ à $C_7$ et

$R^2$ représente un méthyle ou un éthyle, et de leurs sels pharmaceutiquement acceptables, caractérisé en ce qu'on fait réagir un composé de formule

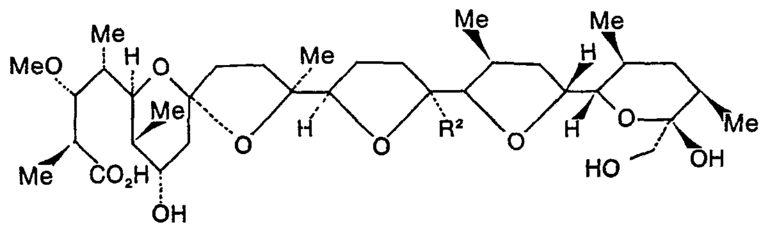

II

où

$R^2$ représente un méthyle ou un éthyle, avec un isocyanate de formule

$$R^1\text{-NCO} \qquad \text{III}$$

où

$R^1$ a la signification donnée ci-dessus.

2. Procédé selon la revendication 1 de préparation de composés où $R^1$ représente un phényle, un alcoyle en $C_1$ à $C_7$-phényle, un halogénophényle, un nitrophényle, un alcoxy en $C_1$ à $C_7$-phényle, un phénoxy-phényle, un alcanoyloxy en $C_1$ à $C_7$-phényle ou un cycloalcoyle en $C_3$ à $C_7$, caractérisé en ce qu'on utilise un isocyanate de formule III substitué de façon correspondante.

3. Procédé selon la revendication 1 de préparation de composés où $R^1$ représente un phényle, un alcoyle en $C_1$ à $C_7$-phényle ou un halogénophényle, caractérisé en ce qu'on utilise un isocyanate de formule III substitué de façon correspondante.

4. Procédé selon l'une des revendications 1–3 de préparation de composés où $R^2$ représente un éthyle, caractérisé en ce qu'on utilise un composé de départ de formule II substitué de façon correspondante.

5. Procédé selon l'une des revendications 1–4 de préparation de composés où $R^1$ représente un phényle ou un halogénophényle, caractérisé en ce qu'on utilise un isocyanate de formule III substitué de façon correspondante.

6. Procédé selon la revendication 5 de préparation de composés où $R^1$ représente un phényle et $R^2$ un éthyle, caractérisé en ce qu'on utilise des composés de départ de formules II et III substitués de façon correspondante.

7. Procédé selon la revendication 5 de préparation de composés où $R^1$ représente un 4-chlorophényle et $R^2$ un éthyle, caractérisé en ce qu'on utilise des composés de départ de formules II et III substitués de façon correspondante.

8. Procédé selon la revendication 5 de préparation de composés où $R^1$ représente un 4-bromophényle et $R^2$ un éthyle, caractérisé en ce qu'on utilise des composés de départ de formules II et III substitués de façon correspondante.

9. Procédé selon la revendication 5 de préparation de composés où $R^1$ représente un 4-fluorophényle et $R^2$ un éthyle, caractérisé en ce qu'on utilise des composés de départ de formules II et III substitués de façon correspondante.

10. Procédé selon la revendication 5 de préparation de composés où $R^1$ représente un 4-nitrophényle et $R^2$ un éthyle, caractérisé en ce qu'on utilise des composés de départ de formules II et III substitués de façon correspondante.

11. Procédé selon l'une des revendications 1 ou 4 de préparation de composés où $R^1$ représente un phénéthyle, caractérisé en ce qu'on utilise un composé de départ de formule II substitué de façon correspondante.

12. Procédé selon l'une des revendications 1–11, caractérisé en ce qu'on utilise l'isocyanate de formule III en léger excès, par exemple de 10%.

13. Procédé selon l'une des revendications 1 ou 4 de préparation de composés où $R^1$ représente un phénéthyle, caractérisé en ce qu'on cultive une sous-culture de l'une des souches Streptomyces sp. X-14667 NRRL 11336 (ATCC 31551), Streptomyces sp. X-14573 NRRL 11337 (ATCC 31552) et Streptomyces sp. X-14575 NRRL 11338 (ATCC 31553) dans une solution aqueuse contenant des hydrates de carbone et des agents nutritifs azotés dans des conditions aérobies submergées puis en ce qu'on isole le produit final à partir de la solution.